(19) Europäisches Patentamt
European Patent Office
Office européen des brevets

(11) EP 4 163 272 A1

(12) **EUROPEAN PATENT APPLICATION**
published in accordance with Art. 153(4) EPC

(43) Date of publication:
**12.04.2023 Bulletin 2023/15**

(21) Application number: **21821267.8**

(22) Date of filing: **01.06.2021**

(51) International Patent Classification (IPC):
***C07D 277/82*** (2006.01) ***C07D 417/12*** (2006.01)
***C07D 513/04*** (2006.01) ***A61K 31/5377*** (2006.01)
***A61K 31/496*** (2006.01) ***A61K 31/4439*** (2006.01)
***A61P 29/00*** (2006.01) ***A61P 9/00*** (2006.01)
***A61P 3/10*** (2006.01) ***A61P 25/18*** (2006.01)
***A61P 25/00*** (2006.01) ***A61P 1/04*** (2006.01)
***A61P 27/02*** (2006.01)

(52) Cooperative Patent Classification (CPC):
**A61K 31/428; A61K 31/4439; A61K 31/496; A61K 31/519; A61K 31/5377; A61P 1/04; A61P 3/10; A61P 9/00; A61P 25/00; A61P 25/18; A61P 27/02; A61P 29/00; C07D 277/82; C07D 417/12; C07D 513/04**

(86) International application number:
**PCT/CN2021/097571**

(87) International publication number:
**WO 2021/249234 (16.12.2021 Gazette 2021/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.06.2020 CN 202010511064**

(71) Applicants:
- **Shenyang Pharmaceutical University**
  **Shenyang, Liaoning 110016 (CN)**
- **Hebei University of Science and Technology**
  **Shijiazhuang, Hebei 050018 (CN)**

(72) Inventors:
- **ZHAO, Yanfang**
  **Shenyang, Liaoning 110016 (CN)**
- **GAO, Zibin**
  **Shijiazhuang, Hebei 050018 (CN)**
- **HAN, Yufei**
  **Shenyang, Liaoning 110016 (CN)**
- **LI, Shuo**
  **Shijiazhuang, Hebei 050018 (CN)**
- **HOU, Yunlei**
  **Shenyang, Liaoning 110016 (CN)**
- **ZHANG, Huimin**
  **Shijiazhuang, Hebei 050018 (CN)**
- **XU, Sicong**
  **Shenyang, Liaoning 110016 (CN)**
- **SUN, Yanping**
  **Shijiazhuang, Hebei 050018 (CN)**
- **QIN, Mingze**
  **Shenyang, Liaoning 110016 (CN)**
- **SUN, Yongjun**
  **Shijiazhuang, Hebei 050018 (CN)**
- **LIU, Yajing**
  **Shenyang, Liaoning 110016 (CN)**
- **GONG, Ping**
  **Shenyang, Liaoning 110016 (CN)**

(74) Representative: **Witte, Weller & Partner Patentanwälte mbB**
**Postfach 10 54 62**
**70047 Stuttgart (DE)**

# (54) BENZOTHIAZOLE DERIVATIVE AND APPLICATION THEREOF

(57) A compound represented by general formula (I) or stereisomers thereof and pharmaceutically acceptable salts, solvates or prodrugs thereof, a preparation method therefor and a pharmaceutical composition containing the compound. An application of the compound of general formula (I) in preparation of drugs for treating and/or preventing she-mediated diseases, especially an application in preparation of drugs for treating inflammatory diseases, cardiovascular and cerebrovascular diseases, diabetes, diabetes complications, diabetes-related dis-

EP 4 163 272 A1

eases, fibrotic diseases, neurological and mental diseases, pain, and ulcer diseases, etc.

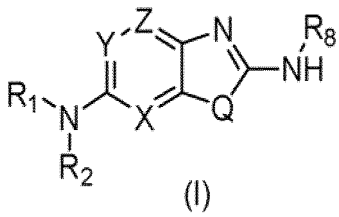

(I)

**Description**

FIELD OF THE INVENTION

The present invention relates to the field of medicinal chemistry, and in particular to a series of novel benzothiazole derivatives and pharmaceutically acceptable salts, solvates or prodrugs thereof, a preparation method for the same, as well as a pharmaceutical composition comprising the compound. The present invention also relates to the use of such compounds in the preparation of a medicament for treating and/or preventing a sEH-mediated disease, and in particular in the preparation of a medicament for treating inflammatory diseases, cardiovascular and cerebrovascular diseases, diabetes, diabetic complications, diabetes-related diseases, fibrotic diseases, neurological and mental diseases, pains, ulcerative diseases, and the like.

BACKGROUND OF THE INVENTION

Epoxide hydrolase (EH) is ubiquitous in mammals, insects, plants and microorganisms, and is an enzyme widely distributed in nature. Currently, epoxide hydrolases are classified into eight types according to their activities, biochemical characteristics, and current research results. Soluble epoxide hydrolase (sEH) is one of the in-depth studied epoxide hydrolase at this stage. It was first discovered in 1973 by Gill *et al.* when they were studying the metabolism of terpenoid epoxides similar to insect juvenile hormones, named for its location in soluble components of the cell such as the cytoplasm and peroxisome. A large number of researches in recent years have shown that the soluble epoxide hydrolase is a potential therapeutic target for many diseases such as hypertension, lung disease, diabetes, pains, inflammation, and other immune system diseases.

Human sEH is encoded by the EPHX2 gene, which is located in the P21-P12 region of human chromosome 8, and consists of 19 exons of 27-265 bp and 18 introns, with a full length of about 45 kb and a total of 555 amino acids encoded. sEH is expressed in almost all organs, and has been found in liver, kidney, lung, heart, brain, spleen, adrenal gland, intestine, bladder, vascular endothelium, smooth muscle, leucocytes, and ovarian cells. It has the highest activity in the liver, followed by the kidney. sEH plays an important role in the metabolism of lipid epoxides. Its endogenous substrates include epoxyeicosatrienoic acids (EETs). EETs are a class of endogenous chemical mediators that have been shown to have a variety of physiological functions, such as dilating blood vessels, lowering blood pressure, regulating lipid metabolism and insulin resistance, etc. (Q. Wang, W. Pang, Z. CuI, American Journal of physiology-Renal physiology, 2013, 304: 168-176). Studies have found that EETs *in vitro* can be rapidly hydrolyzed into dihydroxyeicosatrienoc acids (DHETs) by the action of sEH, thereby losing their activities. Thus, how to inhibit the activity of sEH becomes a key issue. In recent years, studies have shown that the activity of sEH can be reduced by small molecule inhibitors or gene knockout technology to stabilize or increase the content of EETs.

The pharmacological effect obtained by inhibiting sEH can be measured by the level of intracellular EETs. The research on sEH inhibitors mainly focuses on the effects on blood vessels and inflammation. EETs are activators that inhibit nuclear transcription factor (NF-κB). In the animal model of inflammation, the inhibition of sEH can improve and control the inflammation. Furthermore, in the animal model of inflammation, tobacco smoke-induced airway inflammation and monocrotaline-induced pulmonary hypertension were also reduced in severity by inhibiting sEH. sEH inhibitors also have certain analgesic effects. In the animal model experiment of cerebral ischemia, the inhibition of sEH activity can inhibit the ischemic inflammatory response, stimulate the apoptosis of anti-neuronal gene expression, and reduce the effect of cerebral infarction (A. Cautaux, F. Adam, C. J. Willer, Joint Bone Spine, 2005, 72: 359-371).

Studies have found that there is a key aspartic acid residue in the catalytic site of sEH, so the early research on sEH inhibitors mainly focused on modification reagents for carboxyl. Dicyclohexylcarbodiimide (DCC), as a common reagent in the syntheses of amide bonds and peptides, can react with a carboxylic acid to form the corresponding ester. Thus, it is unexpectedly found that DCC has a good inhibitory activity on sEH. However, according to the subsequent pharmacokinetic analysis, DCC will be hydrolyzed in solution into the corresponding urea, which is the main active substance for inhibiting sEH, so most of the early sEH inhibitors contain urea structures (C. Morisseau, B. D. Hammock, Annual Review of Pharmacology and Toxicology, 2005, 45: 311 to 333). t-AUCB is a sEH inhibitor jointly discovered by the University of California and UCD Cancer Center. Studies have found that this compound has a relatively high inhibitory activity on sEH in various mammals and humans.

At present, some compounds with outstanding activities and good therapeutic effects are being in the preclinical and clinical stages, such as AR9281, GSK2256294, and EC5026. EC5026 is a urea-based sEH inhibitor developed by the team of Bruce Hammock from the University of California. This drug is currently undergoing Phase Ia clinical trials, and its indication involves the neuropathic pains.

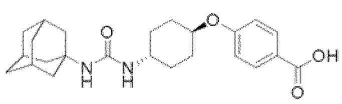

t-AUCB

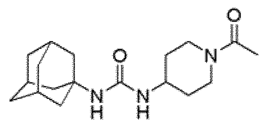

AR9281

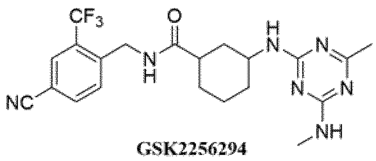

GSK2256294

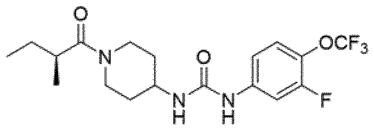

EC5026

On the basis of the references, the inventors have designed and synthesized a series of benzothiazole derivatives, and the results from the *in vitro* sEH kinase inhibitory activity test have shown that they all have inhibitory activities.

SUMMARY OF THE INVENTION

The present invention relates to a compound of general formula I or its stereoisomers as well as pharmaceutically acceptable salts, solvates or prodrugs thereof,

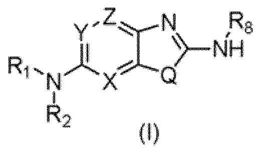

(I)

wherein,

X is CH or N;
Y is CH or N;
Z is CH, N or (one or direct) bond;
Q is O, S, NH or $NCH_3$;
$R_1$ is hydrogen, $-(CH_2)_mNR_3R_4$, $-C(O)NH(CH_2)_nR_5$, $-C(O)(CH_2)_nR_5$ or - $S(O)_2NH(CH_2)_nR_5$:
m is an integer from 2 to 4 (e.g., 2, 3 or 4);
$R_2$ is hydrogen, $-C(O)NH(CH_2)_nR_5$, $-C(O)(CH_2)_nR_5$, $-S(O)_2NH(CH_2)_nR_5$ or - $(CH_2)_mNR_3R_4$; and
$R_1$ and $R_2$ are not hydrogen at the same time;
n is an integer from 0 to 4 (e.g., 0, 1, 2, 3 or 4);
$R_3$ and $R_4$ are same or different, and independently selected from hydrogen, $(C_1-C_6)$alkyl, $(C_3-C_7)$cycloalkyl, $(C_1-C_6)$alkylacyl, $(C_1-C_6)$alkoxyl, $(C_2-C_6)$alkenyl or $(C_2-C_6)$alkynyl; or
$R_3$ and $R_4$ together with the nitrogen atom to which they are attached form a 4 to 10 (e.g., 4, 5, 6, 7, 8, 9 or 10) membered heterocyclic group or 5 to 10 (e.g., 5, 6, 7, 8, 9 or 10) membered heteroaryl group, wherein the heterocyclic group or heteroaryl group optionally contains 0 to 4 (e.g., 0, 1, 2, 3 or 4) heteroatom(s) selected from N, O, and/or S in addition to the nitrogen atom to which $R_3$ and $R_4$ are attached, the heterocyclic group optionally comprises 0 to 2 carbon-carbon double bond(s) or carbon-carbon triple bond(s) in addition to the nitrogen atom to which $R_3$ and $R_4$ are attached, and the heterocyclic group or heteroaryl group is optionally substituted by 1 to 3 (e.g., 1, 2 or 3) same or different $R_6$;
$R_5$ is hydrogen, $(C_1-C_6)$alkyl, $(C_3-C_{10})$cycloalkyl, $(C_6-C_{10})$aryl or 5 to 10 (e.g., 5, 6, 7, 8, 9 or 10) membered heteroaryl group, wherein the heteroaryl group contains 1 to 3 (e.g., 1, 2 or 3) heteroatom(s) selected from N, O or S, and the aryl or heteroaryl group is optionally substituted by 1 to 3 (e.g., 1, 2 or 3) same or different $R_7$;
$R_8$ is hydrogen, $-C(O)(CH_2)_qR_9$, $-(CH_2)_qC(O)OR_9$ or $-C(O)NH(CH_2)_qR_9$;
$R_9$ is $(C_1-C_6)$alkyl, $(C_3-C_7)$cycloalkyl, $(C_6-C_{10})$aryl or 5 to 10 membered (e.g., 5, 6, 7, 8, 9 or 10) heteroaryl group, wherein the aryl or heteroaryl group is optionally substituted by 1 to 3 (e.g., 1, 2 or 3) same or different $R_{10}$;
q is an integer from 0 to 4 (e.g., 0, 1, 2, 3, 4);
$R_6$, $R_7$, and $R_{10}$ are independently 1 to 3 (e.g., 1, 2 or 3) same or different substituent(s) selected from hydrogen, hydroxyl, halogen, nitro, amino, cyano, azido, mercapto, $(C_1-C_6)$alkyl, $(C_1-C_6)$alkoxyl, $(C_1-C_6)$alkyl or $(C_1-C_6)$alkoxyl optionally substituted by hydroxyl, amino or halogen, $(C_1-C_6)$alkylthiol, allyl, amino substituted by mono or di($C_1$-$C_6$

alkyl), ($C_1$-$C_6$)alkylamido, free, salified, esterified, or amidated carboxyl, ($C_1$-$C_6$)alkylsulfinyl, ($C_1$-$C_6$)alkylsulfonyl, ($C_1$-$C_6$)alkylacyl, carbamoyl or carbamoyl substituted by mono or di($C_1$-$C_6$ alkyl).

Preferably, the present invention relates to a compound of general formula I and its stereoisomers as well as pharmaceutically acceptable salts, solvates or prodrugs thereof,

wherein,

Z is CH;

$R_1$ is hydrogen or $-(CH_2)_mNR_3R_4$;

$R_2$ is hydrogen, $-C(O)NH(CH_2)_nR_5$ or $-C(O)(CH_2)_nR_5$;

$R_1$ and $R_2$ are not hydrogen at the same time;

$R_3$ and $R_4$ are same or different, and independently selected from hydrogen, ($C_1$-$C_6$)alkyl, ($C_3$-$C_7$)cycloalkyl, ($C_1$-$C_6$)alkylacyl, ($C_1$-$C_6$)alkoxyl, ($C_2$-$C_6$)alkenyl or ($C_2$-$C_6$)alkynyl; or

$R_3$ and $R_4$ together with the nitrogen atom to which they are attached form a 4 to 10 membered heterocyclic group, wherein the heterocyclic group optionally contains 1 to 4 heteroatom(s) selected from N, O, and/or S in addition to the nitrogen atom to which $R_3$ and $R_4$ are attached, and optionally substituted by 1 to 3 same or different $R_6$.

More preferably, the present invention relates to a compound of general formula I and its stereoisomers as well as pharmaceutically acceptable salts, solvates or prodrugs thereof, wherein

$R_5$ is ($C_3$-$C_{10}$)cycloalkyl, phenyl or a 5 to 10 membered heteroaryl group containing 1 to 3 heteroatom(s) selected from N, O or S; more preferably ($C_3$-$C_7$)cycloalkyl, phenyl, indolyl, quinolyl, pyridyl, pyrimidinyl, furyl, thienyl, pyrrolyl, wherein aryl or heteroaryl group is optionally substituted by 1 to 3 same or different $R_7$;

More preferably, the present invention relates to a compound of general formula I and its stereoisomers as well as pharmaceutically acceptable salts, solvates or prodrugs thereof,

wherein,

$R_1$ is hydrogen or $-(CH_2)_mNR_3R_4$;

$R_3$ and $R_4$ are same or different, and independently selected from hydrogen, ($C_1$-$C_6$)alkyl or ($C_3$-$C_7$)cycloalkyl or together with the nitrogen atom to which they are attached form

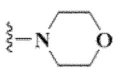, 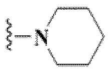, 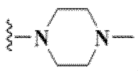, 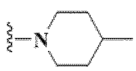, 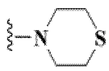, 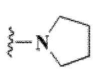, 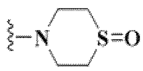 or 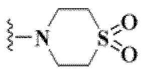;

$R_2$ is hydrogen, $-C(O)NH(CH_2)_nR_5$ or $-C(O)(CH_2)_nR_5$,

Particularly preferably, the present invention relates to a compound of general formula I and its stereoisomers as well as pharmaceutically acceptable salts, solvates or prodrugs thereof,

wherein,

$R_9$ is phenyl optionally substituted by 1 to 3 same or different $R_{10}$.

Particularly preferably, the present invention relates to the following compound of general formula I and its stereoisomers as well as pharmaceutically acceptable salts, solvates or prodrugs thereof:

1-(2-aminobenzo[*d*]thiazol-6-yl)-3-(4-chlorophenyl)urea
1-(2-aminobenzo[*d*]|thiazol-6-yl)urea
1-(2-aminobenzo[*d*]thiazol-6-yl)-3-phenylurea
1-(2-aminobenzo[*d*]thiazol-6-yl)-3-[(3s,5s,7s)-adamantan-1-yl]urea
1-(2-aminobenzo[*d*]thiazol-6-yl)-3-(4-chlorobenzyl)urea
1-(2-aminobenzo[*d*]thiazol-6-yl)-3-cyclohexylurea
1,1'-(benzo[*d*]thiazol-2,6-diyl)bis[3-(4-chlorophenyl)urea]
1-(2-aminobenzo[*d*]thiazol-6-yl)-1-[2-(4-morpholinyl)ethyl]-3-(4-nitrophenyl)urea
1-(2-aminobenzo[*d*]thiazol-6-yl)-1-[2-(4-morpholinyl)ethyl]-3-(4-acetylphenyl)urea
1-(2-aminobenzo[*d*]thiazol-6-yl)-1-[2-(4-morphotinyl)ethyl]-3-(4-acetatnidophenyl)urea
*N*-(2-aminobenzo[*d*]thiazol-6-yl)-2-(4-chlorophenyl)-*N*-[2-(4-morpholinyl)ethyl] acetamide
1-(2-aminobenzo[*d*]thiazol-6-yl)-1-[2-(4-morpholinyl)ethyl]-3-(4-chlorophenyl)urea
1-(2-aminobenzo[*d*]thiazol-6-yl)-1-[2-(4-morpholinyl)ethyl]-3-(4-trifluoromethylphenyl)urea
1-(2-aminobenzo[*d*]thiazol-6-yl)-1-[2-(4-morpholinyl)ethyl]-3-(pyridin-2-yl)urea
1-(2-aminobenzo[*d*]thiazol-6-yl)-1-[2-(4-morpholinyl)ethyl]-3-(naphthalen-1-yl)urea
1-(2-aminobenzo[*d*]thiazol-6-yl)-1-[2-(4-morpholinyl)ethyl]-3-(3-bromo-4-fluorophenyl)urea
1-(2-aminobenzo[*d*]thiazol-6-yl)-1-[2-(4-morpholinyl)ethyl]-3-(4-trifluoromethoxyphenyl)urea
1-(2-aminobenzo[*d*]thiazol-6-yl)-1-[2-(4-morpholinyl)ethyl]-3-(4-ethylphenyl)urea
1-(2-aminobenzo[*d*]thiazol-6-yl)-1-[2-(4-morpholinyl)ethyl]-3-(4-isopropoxyphenyl)urea

1-(2-aminobenzo[*d*]thiazol-6-yl)-1-[2-(4-morpholinyl)ethyl]-3-{4-isopropylphenyl)urea
1-(2-aminobenzo[*d*]thiazol-6-yl)-1-[2-(*N*,*N*-diethylamino)ethyl]-3-(4-chlorophenyl)urea
1-(2-aminobenzo[*d*]thiazol-6-yl)-1-[2-(4-methylpiperidin-1-yl)ethyl]-3-(4-chlorophenyl)urea
1-(2-aminobenzo[*d*]thiazol-6-yl)-1-[2-(4-methylpiperazin-1-yl)ethyl]-3-(4-trifluoromethylphenyl)urea
1-(2-aminobenzo[*d*]thiazol-6-yl)-1-[2-(pyrrolidin-1 -yl)ethyl]-3-(4-chlorophenyl)urea
1-(2-aminobenzo[*d*]thiazol-6-yl)-1-[2-(*N*,*N*-dimethylamino)ethyl]-3-(4-chlorophenyl)urea
1-(2-aminobenzo[*d*]thiazol-6-yl)-1-[3-(4-morpholinyl)propyl]-3-(4-chlorophenyl)urea
1-(2-phenylacetamidobenzo[*d*]thiazol-6-yl)-1-[2-(4-morpholinyl)ethyl]-3-(4-chlorophenyl)urea
1-[2-(2-ethoxyformamido)benzo[*d*]thiazot-6-yl]-1-[2-(4-morpholinyl)ethyl]-3-(4-chlorophenyl)urea
1-{[2-(2-ethoxyformyl)ethylamino-1-yl]benzo[*d*]thiazol-6-yl}-1-[2-(4-morpholinyl)ethyl]-3-(4-chlorophenyl)urea
1-(2-acetamidobenzo[*d*]thiazol-6-yl)-1-[2-(4-morpholinyl)ethyl]-3-(4-chlorophenyl)urea
Tert-butyl(6-{2-(4-chlorophenyl)-*N*-[2-(4-morpholinyl)ethyl]acetamido}benzo[*d*]thiazol-2-yl)carbamate
1-(2-phenylformamidobenzo[*d*]thiazol-6-yl)-1-[2-(4-morpholinyl)ethyl]-3-(4-chlorophenyl)urea
1-(2-phenylpropionamidobenzo[*d*]thiazol-6-yl)-1-[2-(4-morpholinyl)ethyl]-3-(4-chlorophenyl)urea
1-(2-phenylacetamidobenzo[*d*]thiazol-6-yl)-1-[2-(4-morpholinyl)ethyl]-3-(4-trifluoromethylphenyl)urea
1-(2-aminobenzo[*d*]oxazol-6-yl)-1-[2-(4-morpholinyl)ethyl]-3-(4-methylphenyl)urea
1-(2-acetamidobenzo[*d*]oxazol-6-yl)-1-[2-(4-morpholinyl)ethyl]-3-(4-methylphenyl)urea
1-(2-phenylacetamidobenzo[*d*]oxazol-6-yl)-1-[2-(4-morpholinyl)ethyl]-3-(4-methylphenyl)urea
1-(2-aminothiazo[5,4-*b*]pyridin-5-yl)-3-(4-fluorophenyl)-1-[2-(4-morpholinyl)ethyl]urea
1-[2-(ethylamino)thiazo[5,4-*b*]pyridin-5-yl]-1-[2-(4-morpholinyl)ethyl]-3-(4-fluorophenyl)urea
1-{2-[3-(4-chlorophenyl)ureido]benzo[*d*]thiazol-6-yl}-1-[2-(4-morpholinyl)ethyl]-3-(4-chlorophenyl)urea
1-(2-aminothiazo[4,5-*c*]pyridin-6-yl)-1-[2-(4-morpholinyl)ethyl]-3-(4-trifluoromethoxyphenyl)urea
1-(2-cyclopropylformamidothiazo[4,5-*c*]pyridin-6-yl)-1-[2-(4-morpholinyl)ethyl]-3-(4-trifluoromethoxyphenyl)urea
1-{[2-(2-ethoxyformyl)ethylamino-1-yl]thiazo[4,5-*c*]pyridin-6-yl} -1 -[2-(4-morpholinyl)ethyl]-3-(4-trifluoromethoxyphenyl)urea
1-(2-aminothiazo[5,4-*d*]pyrimidin-5-yl)-1-[2-(4-morpholinyl)ethyl]-3-(pyridin-3-yl)urea
1-(2-acetamidothiazo[5,4-*d*]pyrimidin-5-yl)-1-[2-(4-morpholinyl)ethyl]-3-(pyridin-3-yl)urea
1-(2-phenylpropionamidothiazo[5,4-*d*]pyrimidin-5-yl)-1-[2-(4-morpholinyl)ethyl]-3-(pyridin-3-yl)urea
1-[2-(3-benzylureido)thiazo[4,5-*b*]pyridin-6-yl]-1-[2-(4-morpholinyl)ethyl]-3-(4-bromo-3-fluorophenyl)urea

According to some general methods in the field of the present invention, the compound of general formula I of the present invention can form its pharmaceutically acceptable salt with an acid. The preferred acid is hydrochloric acid, hydrobromic acid, hydrofluoric acid, sulfuric acid, phosphoric acid, nitric acid, formic acid, acetic acid, propionic acid, oxalic acid, malonic acid, succinic acid, fumaric acid, maleic acid, lactic acid, malic acid, tartaric acid, citric acid, picric acid, methanesulfonic acid, ethanesulfonic acid, toluenesulfonic acid, benzenesulfonic acid, naphthalenesulfonic acid, trifluoroacetic acid, and aspartic acid.

The present invention also includes the prodrugs of the derivatives of the present invention. The prodrugs of the derivatives of the present invention are derivatives of general formula (1), which themselves may have a weaker activity or even no activity, but can be converted into the corresponding biologically active forms under physiological conditions (for example, by metabolism, solvolysis or other means) upon administration.

Unless otherwise indicated, as used in the present invention, the term "halogen" refers to fluoro, chloro or bromo; the term "alkyl" refers to linear or branched alkyl; the term "cycloalkyl" refers to substituted or unsubstituted cycloalkyl; the term "alkoxyl" refers to linear or branched alkoxyl; the term "alkenyl" refers to linear or branched alkenyl; the term "alkynyl" refers to linear or branched alkynyl; the term "aryl" refers to a phenyl group with or without substituent(s); the term "heteroaryl group"refers to a monocyclic or polycyclic ring system which contains one or more heteroatom(s) selected from N, O, and S, and which is aromatic, such as imidazolyl, pyridyl, pyrazolyl, furyl, thienyl, pyrrolyl, thiazolyl, benzothiazolyl, oxazolyl, isoxazolyl, naphthyl, quinolyl, isoquinolyl, benzimidazolyl, and benzoxazolyl.

The present invention also includes a pharmaceutical composition comprising the compound of general formula I and pharmaceutically acceptable salts and/or solvates thereof as active ingredient as well as a pharmaceutically acceptable carrier. The compound of the present invention can be used in combination with other active ingredients as long as they do not bring about other adverse effects such as allergic reactions.

The carrier used in the pharmaceutical composition of the present invention is a common type available in the pharmaceutical field, including: adhesives, lubricants, disintegrants, cosolvents, diluents, stabilizers, suspending agents, pigments, flavoring agent, and the like for oral preparations; pH regulators, osmotic pressure regulators, solubilizers, stabilizers, and the like for injection preparations; and substrates, diluents, lubricants, preservatives, and the like for topical preparations. The pharmaceutical preparations may be administrated orally, parenterally (e.g., intravenously, subcutaneously, intraperitoneally, etc.) or topically (e.g., ocularly, nasally, sublingually, transdermally, etc.). If a certain drug is unstable under stomach conditions, it may be formulated into an enteric-coated tablet.

Through *in* vivo and *in vitro* inhibitory activity tests, it has been found that the compound of the present invention has a significant inhibitory activity on sEH, and can stabilize the level of EETs *in vivo*. The results of pharmacodynamic studies have shown that the compound of the present invention can exert the functions such as inhibiting the paw swelling and pain induced by carrageenan as well as the release of inflammatory factors induced by lipopolysaccharide, protecting the traumatic brain injury, maintaining the blood glucose homeostasis, improving the diabetic retinopathy, resisting against the tissue fibrosis, and relieving the depression, and thus can be used to treat sEH-mediated inflammatory diseases, cardiovascular and cerebrovascular diseases, diabetes and complications thereof, fibrotic diseases, neurological and mental diseases, pains, ulcerative diseases, etc.

The precise dosage of a compound of the present invention required for treating the sEH-mediated condition will vary from subject to subject, depending on the species, age, and general conditions of the subject, the severity of the disease to be treated, the particular compound used, and the administration manner (such as the administration route and frequency), etc. An appropriate effective amount can be determined by one of ordinary skill in the art through conventional experimentation.

The dosage of the compound may be about 0.1-100 mg/kg body weight/day, preferably 1-50 mg/kg body weight/day. It will be appreciated that the dosage may vary depending on the requirements of the patient, the severity of the hypertensive, cardiovascular, inflammatory, and diabetes-related diseases to be treated, and the particular compound employed. Furthermore, it will be appreciated that the initial dosage administrated may be increased beyond the upper limit in order to rapidly achieve the desired blood level or the initial dosage may be less than the optimal value. The daily dosage may be gradually increased during treatment, depending on the specific conditions. If necessary, the daily dosage may also be divided into multiple doses, for example 2-4 times a day.

The mammals mean humans or animals.

The amount of the active ingredient (i.e., the compound according to the present invention) in the pharmaceutical composition and its unit dosage form may vary depending on the particular application, the potency and desired concentration of the particular compound. Generally, the content of the active ingredient will be between 0.5% and 90%, based on the total weight of the composition.

In the combination therapy, the compound of the present invention and other compound(s) may be administrated simultaneously or at intervals. When administrated simultaneously, the compound of the present invention and other compound(s) may be combined in a single pharmaceutical composition or in separate compositions.

The compounds of the present invention and preparation methods thereof are further explained and illustrated in the Examples and preparations provided hereinafter. It should be understood that the scope of the following Examples and preparations shall not limit the scope of the present invention in any way.

The following synthetic routes describe the preparations for the derivatives of general formula I in the present invention. All starting materials are prepared by the methods described in these synthetic routes or by the methods well known to one of ordinary skill in the field of organic chemistry or are commercially available. The final compounds in the present invention are all prepared by the methods described in these synthetic routes or by the methods analogous thereto, which are well known to one of ordinary skill in the field of organic chemistry. All the variables applicable in these synthetic routes are as defined below or as defined in the claims.

According to the compound of general formula I in the present invention, in Routes 1 to 6, the following compounds are exemplified, wherein the definitions for substituents $R_1$, $R_5$, $R_9$, X, Y, Z, etc. are the same as those in the claims.

In Route 1, using 2-amino-6-nitrobenzothiazole as the starting material, DMAP as the base, and Boc anhydride for Boc protection, Intermediate B is obtained, followed by reduction reaction under the condition of hydrazine hydrate and activated carbon to obtain Intermediate C, followed by substitution reaction with phenyl chloroformate to obtain Intermediate D, followed by nucleophilic substitution reaction with $R_5$-substituted amine to obtain Intermediate E, followed by Boc deprotection reaction with trifluoroacetic acid, to generate Compound 1-i of general formula I.

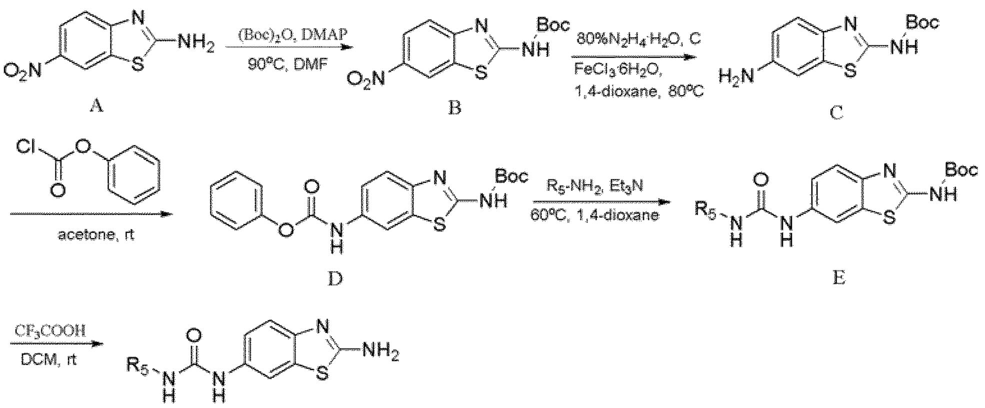


I-i

**Route 1 Synthesis of compound I-i**

In Route 2, Intermediate C is subjected to a nucleophilic substitution reaction with $R_1$-substituted chlorinated compound under the condition of potassium carbonate as the base to obtain Intermediate F, followed by nucleophilic substitution reaction with $R_5$-substituted phenyl chloroformate G to obtain Intermediate H, followed by Boc deprotection reaction under the condition of trifluoroacetic acid, to generate Compound I-ii of general formula I.

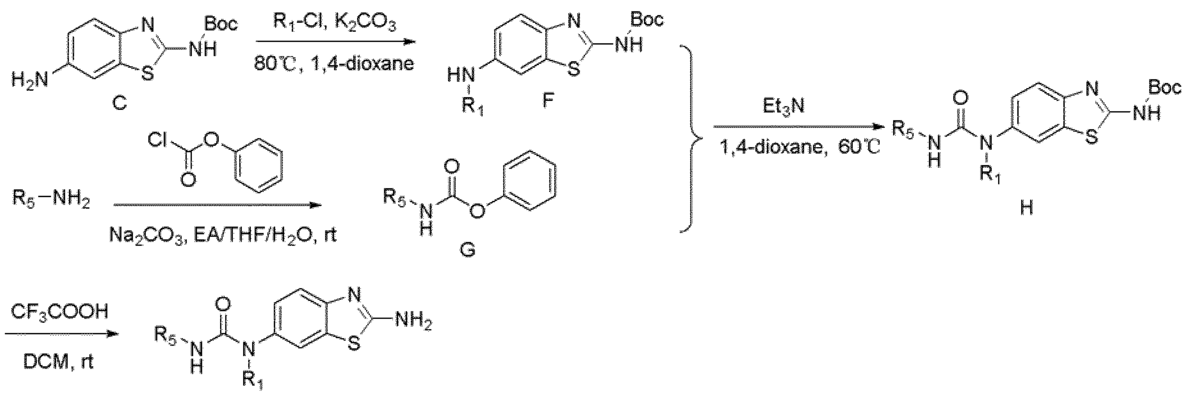


I-ii

**Route 2 Synthesis of compound I-ii**

In Route 3, Compound I-ii and $R_9$-substituted chlorinated compound are used to prepare Compound I-iii of general formula I under the condition of triethylamine as acid-binding agent; or Compound I-ii and $R_9$-substituted phenyl chloroformate are used to generate urea under the condition of DIPEA as the base, to prepare Compound I-iv of general formula I.

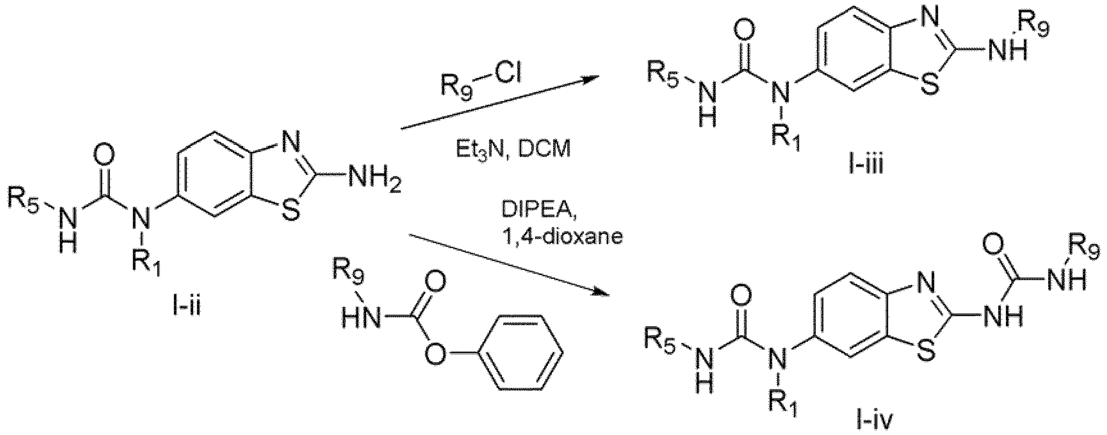

**Route 3 Synthesis of compounds I-iii and 1-iv**

In Route 4, using 2-amino-5-nitrophenol (J) as the starting material, and a solution of boron trifluoride in diethyl ether is used for a tube-sealed reaction, Intermediate K is obtained, followed by reduction reaction under the condition of hydrogen and palladium on carbon to obtain Intermediate L, followed by nucleophilic substitution reaction with $R_1$-substituted chlorinated compound under the condition of potassium carbonate as the base to obtain Intermediate M, followed by nucleophilic substitution reaction with $R_5$-substituted phenyl chloroformate G to prepare Compound I-v. Compound I-v and $R_9$-substituted chlorinated compound are used to prepare Compound I-vi of general formula I under the condition of triethylamine as acid-binding agent; or Compound I-v and $R_9$-substituted phenyl chloroformate are used to generate urea under the condition of DIPEA as the base, to prepare Compound I-vii of general formula I.

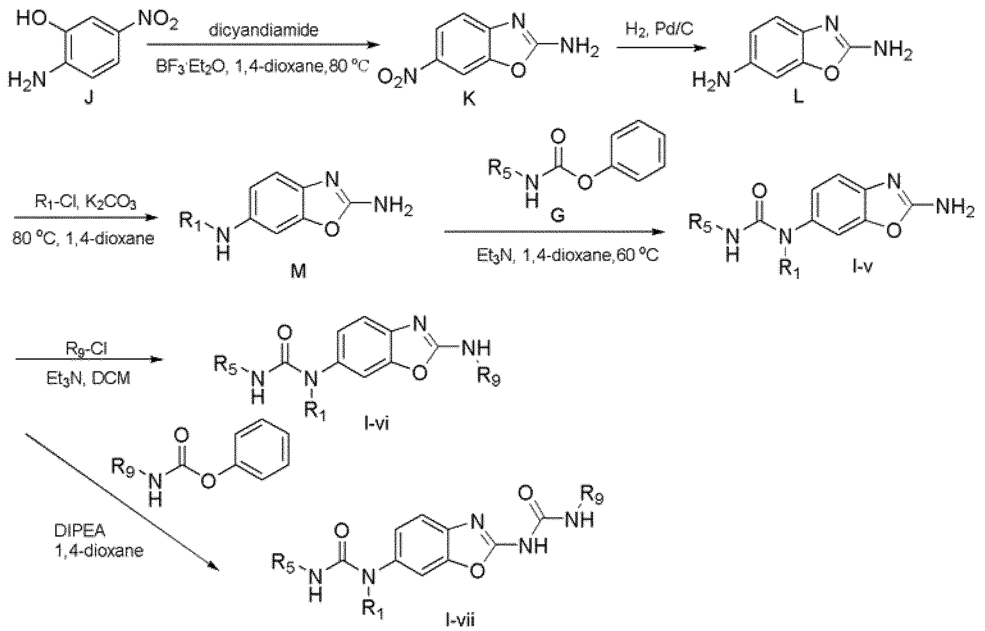


**Route 4 Synthesis of compounds I-v, 1-vi, and I-vii**

In Route 5, 2-chloro-5-aminopyridine (N) is used as the starting material and reacted with potassium thiocyanate and bromine, to obtain Intermediate O, which is subjected to nucleophilic substitution reaction with sodium hydroxide as the base to obtain Intermediate P, followed by nucleophilic substitution reaction with $R_5$-substituted phenyl chloroformate G, to prepare Compound I-viii. Compound I-viii and $R_9$-substituted chlorinated compound are used to prepare Compound I-ix of general formula I under the condition of triethylamine as acid-binding agent; or Compound I-viii and $R_9$-substituted phenyl chloroformate are used to generate urea under the condition of DIPEA as the base, to prepare Compound I-x of general formula I.

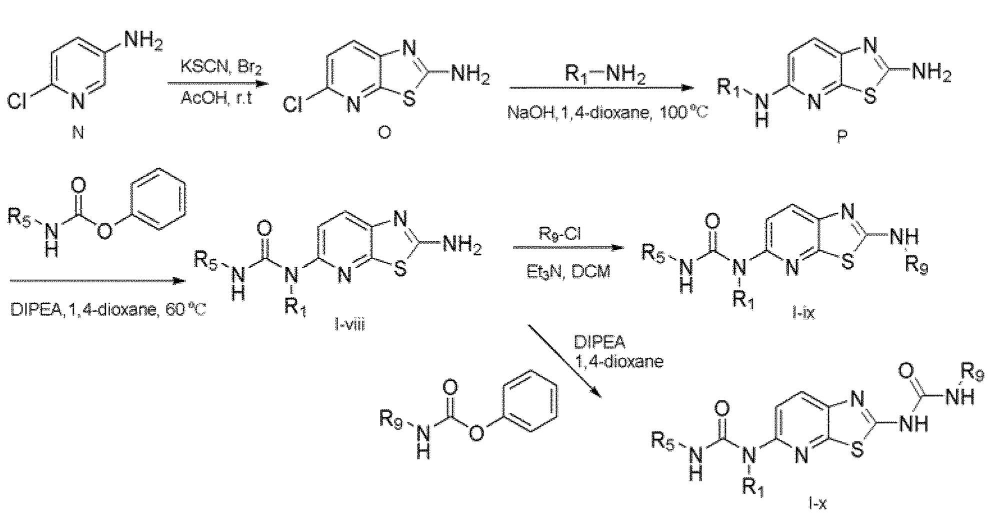

**Route 5 Synthesis of compounds I-viii, I-vix, and I-x**

In Route 6, 4,6-dichloropyridin-3-amine, 2,4-dichloropyrimidin-5-amine or 3,5-dichloropyridin-2-amine (Q) is used as the starting material and subjected to substitution reaction with phenyl isothiocyanate, to obtain Intermediate R; Intermediate R is reacted under the condition of sodium methoxide as the base, to obtain Intermediate S; Intermediate S is reacted in a 70% sulfuric acid solution, to obtain Intermediate T; Intermediate T is subjected to Buchward-Hartwig reaction with $R_1$-substituted primary amine, to obtain Intermediate U; and then Intermediate U is subjected to nucleophilic substitution reaction with $R_5$-substituted phenyl chloroformate G, to prepare Compound I-xi. Compound I-xi and $R_9$-substituted chlorinated compound are used to prepare Compound I-xii of general formula I under the condition of triethylamine as acid-binding agent.

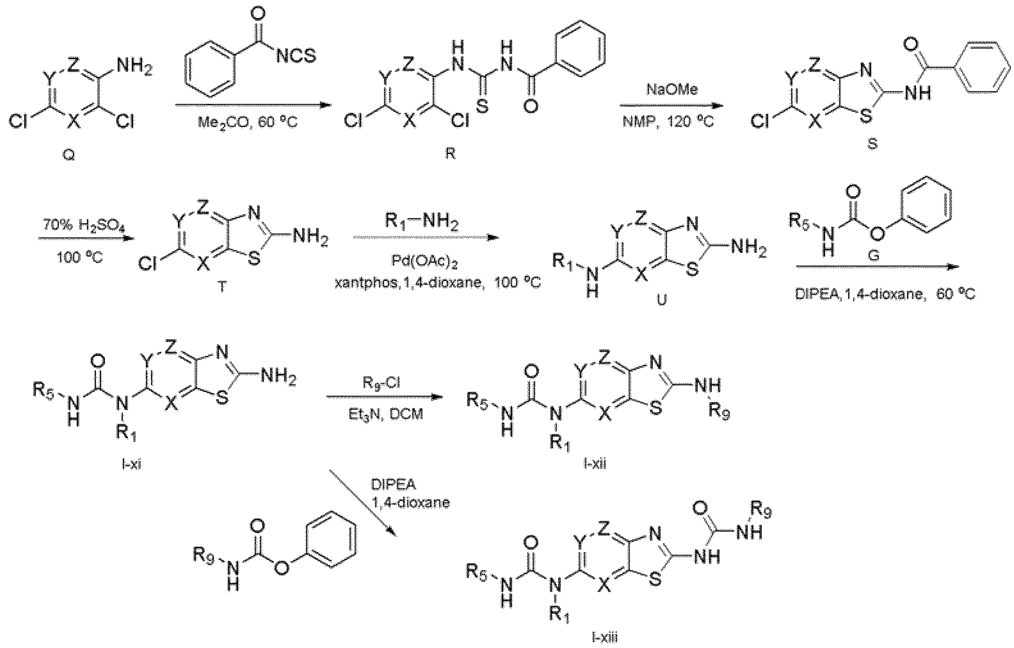


**Route 6 Synthesis of compounds I-xi, I-xii, and I-xiii**

BRIEF DESCRIPTION OF THE DRAWINGS

Fig. 1 The results for the effect of the compound of Example 16 on maintaining the level of EETs *in vivo*
#: Compared with the sham group, $p$<0.05; *: Compared with the UUO group, $p$<0.05.
Fig. 2 The results for the effect of the compound of Example 16 against renal fibrosis #: Compared with the sham group, $p$<0.05; *: Compared with the UUO group, $p$<0.05.
Fig. 3 The results for the anti-inflammatory effects of the compounds of Example 12, 18, 27, and 40
*: Compared with the model group, $p < 0.05$.
Fig. 4 The results for the hypoglycemic effect of the compound of Example 12
*: Compared with the model group, $p < 0.05$.
Fig. 5 The results for the improvements on retinal capillary integrity and permeability of the compound of Example 40
*: Compared with the model group, $p < 0.05$.
Fig. 6 The results for the analgesic effect of the compounds of Examples 12 and 40
*: Compared with the model group, $p < 0.05$.
Fig. 7 The results for the antidepressant effect of the compounds of Examples 12 and 40
*: Compared with the blank solvent group, $p < 0.05$.

DETAILED DESCRIPTION OF THE INVENTION

In the following Examples, the methods for preparing some of said compounds are described. It should be understood that the following methods and other methods known to one of ordinary skill in the art can be applied to the

preparations of all compounds described herein. The Examples are intended to illustrate the scope of the invention, rather than limiting the scope. The proton nuclear magnetic resonance spectrum for the compound was determined by Bruker ARX-400 or Bruker ARX-600, and the mass spectrum was determined by Agilent 1100 LC/MSD; and the reagents used were analytical or chemically pure.

**Example 1 Preparation of 1-(2-aminobenzo[*d*]thiazol-6-yl)-3-(4-chlorophenyl)urea**

**1.1 Synthesis of tert-butyl (6-nitrobenzo[*d*]biazol-2-yl)carbamate (B)**

To a solution of 2-amino-6-nitrobenzothiazole (A) (purchased from Shanghai Bide Pharmatech Ltd.) (10 g, 51.2 mmol) and 4-dimethylaminopyridine (purchased from Beijing Ouhe Technology Co., Ltd.) (3.2 g, 26.2 mmol) in DMF (100 mL), di-tert-butyl dicarbonate (purchased from Shanghai Bide Pharmatech Ltd.) (16.8 g, 77.0 mmol) was added, and the resulting solution was stirred at 90 °C for 18 h. The resulting mixture was poured into stirred ice water (500 mL), then the yellow solid was filtered and dried under reduced pressure. The crude product was purified by flash column chromatography, to obtain 15.3 g of Intermediate B. Yield: 85%. MS (ESI) m/z: 296.3 $[M+H]^+$.

**1.2 Synthesis of tert-butyl (6-aminobenzo[*d*]thiazol-2-yl)carbamate (C)**

Intermediate B (12.0 g, 40.5 mmol) was dissolved in 1,4-dioxane (120 mL), and $FeCl_3•H_2O$ (purchased from Shenyang Aiketongsheng Chemical Reagent Co., Ltd.) (1.0 g, 6.2 mmol), activated carbon (purchased from Shenyang Aiketongsheng Chemical Reagent Co., Ltd.) (0.15 g, 12.5 mmol), and 80% hydrazine hydrate (purchased from Shenyang Aiketongsheng Chemical Reagent Co., Ltd.) (35 mL) were added to the solution, respectively. The mixture solution was heated to 90 °C for 12 h. The mixture solution was filtered through celite, the filter cake was washed with 1,4-dioxane (100 mL), and the filtrate was concentrated under vacuum, to obtain 9.3 g of Intermediate C. Yield: 73.3%. MS (ESI) m/z: 266.2 $[M+H]^+$.

**1.3 Synthesis of tert-butyl phenyl benzo[*d*]thiazol-2,6-dicarbamate (D)**

Intermediate C (10 g, 37.7 mmol) was dissolved in acetone (100 mL), and phenyl chloroformate (purchased from Shanghai Bide Pharmatech Ltd.) (9.0 g, 57.7 mmol) was dropwise added to the solution under stirring. After dropwise addition, the reaction solution was stirred at room temperature for 2 h. The reaction mixture solution was filtered by suction, to obtain 9.4 g of Intermediate D. Yield: 83.6%. MS (ESI) m/z: 386.1 $[M+H]^+$.

**1.4 Synthesis of tert-butyl (6-(3-(4-chlorophenyl)ureido)benzo[*d*]thiazol-2-yl)carbamate (E)**

Intermediate D (0.5 g, 1.3 mmol) was dissolved in 1,4-dioxane (5 mL), and p-chloroaniline (purchased from Beijing Ouhe Technology Co., Ltd.) (0.15 g, 1.5 mmol) and triethylamine (purchased from Shenyang Aiketongsheng Chemical Reagent Co., Ltd.) (0.13 g, 1.3 mmol) were added to the solution. The mixture was heated to 60 °C and stirred for 3 h. The reaction solution was filtered by suction, and the filter cake was washed with diethyl ether, and dried, to obtain 0.3 g of Intermediate E. Yield: 86.1%.

**1.5 Preparation of 1-(2-aminobenzo[*d*]thiazol-6-yl)-3-(4-chlorophenyl)urea (I)**

Intermediate E (0.3 g) was dissolved in dichloromethane (3 mL), and trifluoroacetic acid (purchased from Reagent Station of Shenyang Pharmaceutical University) (3 mL) was added to the solution. After stirring overnight at room temperature, the mixture solution was concentrated under reduced pressure and water was added (5 mL), and filtered by suction after adjusting the pH to 8 with saturated sodium bicarbonate solution. The filter cake was further purified by silica-gel column chromatography, to obtain 0.15 g of Intermediate I. Yield: 77%.

**Example 2 1-(2-aminobenzo[*d*]thiazol-6-yl)urea**

Using sodium cyanate (purchased from Shanghai Bide Pharmatech Ltd.) as raw material, the key Intermediate E was synthesized in accordance with the synthesis process of step 1.4 in Example 1, then 1-(2-aminobenzo[*d*]thiazol-6-yl)urea was synthesized in accordance with the synthesis process of step 1.5 in Example 1. Yield: 65%.

**Example 3 1-(2-aminobenzo[*d*]thiazol-6-yl)-3-phenylurea**

Using aniline (purchased from Beijing Ouhe Technology Co., Ltd.) as raw material, the key Intermediate E was

synthesized in accordance with the synthesis process of step 1.4 in Example 1, then 1-(2-aminobenzo[*d*]thiazol-6-yl)-3-phenylurea was synthesized in accordance with the synthesis process of step 1.5 in Example 1. Yield: 70%.

**Example 4 1-(2-aminobenzo[*d*]thiazol-6-yl)-3-[(3s,5s,7s)-adamantan-1-yl)urea**

Using adamantanamine (purchased from Shanghai Bide Pharmatech Ltd.) as raw material, the key Intermediate E was synthesized in accordance with the synthesis process of step 1.4 in Example 1, then 1-(2-aminobenzo[*d*]thiazo-6-yl)-3-[(3s,5s,7s)-adamantan-1-yl]urea was synthesized in accordance with the synthesis process of step 1.5 in Example 1. Yield: 68%.

**Example 5 1-(2-aminobenzo[*d*]thiazol-6-yl)-3-(4-chlorobenzyl)urea**

Using p-chlorobenzylamine (purchased from Shanghai Bide Pharmatech Ltd.) as raw material, the key Intermediate E was synthesized in accordance with the synthesis process of step 1.4 in Example 1, then 1-(2-aminobenzo[*d*]thiazol-6-yl)-3-(4-chlorobenzyl)urea was synthesized in accordance with the synthesis process of step 1.5 in Example 1. Yield: 54%.

**Example 6 1-(2-aminobenzo[*d*]thiazol-6-yl)-3-cyclohexylurea**

Using cyclohexylamine (purchased from Beijing Ouhe Technology Co., Ltd.) as raw material, the key Intermediate E was synthesized in accordance with the synthesis process of step 1.4 in Example 1, then 1-(2-aminobenzo[*d*]thiazol-6-yl)-3-cyclohexylurea was synthesized in accordance with the synthesis process of step 1.5 in Example 1. Yield: 89%.

**Example 7 1,1'-(benzo[*d*]thiazol-2,6-diyl)bis[3-(4-chlorophenyl)urea]**

1-(2-Aminobenzo[*d*]thiazol-6-yl)-3-(4-chlorophenyl)urea (0.2 g, 0.4 mmol) was dissolved in 1,4-dioxane (3 mL), and DIPEA (purchased from Shanghai Bide Pharmatech Ltd.) (0.1 g, 0.7 mmol) was added. At room temperature, phenyl (4-chlorophenyl)carbamate (0.1 g, 0.4 mmol) was added to the solution, and stirred overnight at room temperature. After the reaction was completed, water (10 mL) was added, and a solid was precipitated, which was filtered by suction. The filter cake was washed, dried, and purified by column chromatography, to obtain 0.08 g of white solid. Yield: 76%.

**Example 8 1-(2-aminobenzo[*d*]thiazol-6-yl)-1-[2-(4-morpholinyl)ethyl]-3-(4-nitrophenyl)urea**

**8.1 Synthesis of tert-butyl (6-{[2-(4-morpholinyl)ethyl]amino}benzo[*d*]thiazol-2-yl)carbamate (F)**

Intermediate C (2.0 g, 7.5 mmol) was dissolved in 1.4-dioxane (20 mL), and potassium carbonate (purchased from Shenyang Aiketongsheng Chemical Reagent Co., Ltd.) (3.1 g, 22.4 mmol) and 4-(2-chloroethyl)morpholine (purchased from Shanghai Bide Pharmatech Ltd.) (1.2 g, 11.2 mmol) were added to the solution. The reaction solution was heated to reflux and reacted under stirring for 8 h. After the reaction was cooled to room temperature, water (80 mL) was added thereto, and extracted with ethyl acetate (2×80 mL). The organic layers were combined, washed with brine, dried, concentrated and dried under reduced pressure, to obtain 1.5 g of Intermediate F. Yield: 60%.

**8.2 Synthesis of phenyl N-(4-chlorophenyl)carbamate (G)**

At room temperature, 4-chloroaniline (purchased from Beijing Ouhe Technology Co., Ltd.) (1.4 g, 11.2 mmol) and sodium carbonate (purchased from Shenyang Aiketongsheng Chemical Reagent Co., Ltd.) (0.7 g, 6.7 mmol) were added to a mixture solution of ethyl acetate-tetrahydrofuran-water (3:1:1=10 mL), and a solution of phenyl chloroformate (purchased from Shanghai Bide Pharmatech Ltd.) (1.9 g, 12.3 mmol) in ethyl acetate (3 mL) was dropwise added in ice-bath. After dropwise addition, the system was reacted at room temperature for 2 h. After the reaction was completed, the reaction solution was concentrated under reduced pressure, and water (10 mL) was added to the residue. After adjusting the pH to 5 with 1 M hydrochloric acid solution, the solution was filtered by suction. The filter cake was washed with water (10 mL), and dried, to obtain 2.5 g of white solid. Yield: 91%.

**8.3 Synthesis of tert-butyl (6-{3-(4-chlorophenyl)-1-12-(4-morpholinyl)ethyl)ureido}benzo[*d*]thiazol-2-yl)carbamate (H)**

At room temperature, Intermediate F (0.5 g, 0.6 mmol) and Intermediate G (0.4 g, 0.7 mmol) were added to

1,4-dioxane (4 mL), and DIPEA (purchased from Shanghai Bide Pharmatech Ltd.) (0.3 g, 2.3 mmol) was dropwise added to the reaction solution. After dropwise addition, the system was heated to 60 °C and reacted for 3 h. After the reaction was completed, the solution was cooled to room temperature, and the solvent was concentrated under reduced pressure. To the residue water (10 mL) was added. After adjusting the pH to 10 with 2.5% NaOH solution, the solution was filtered by suction, and dried, to obtain a crude product. The crude product was purified by column chromatography (dichloromethane:methanol=20:1), to obtain 0.35 g of white solid. Yield: 62%.

**8.4 Preparation of 1-(2-aminobenzo[*d*]thiazol-6-yl)-3-(4-chlorophenyl)-1-[2-(4-morpholinyl)ethyl]urea(I)**

Intermediate H (0.3 g, 0.6 mmol) was dissolved in dichloromethane (3 mL), and trifluoroacetic acid (purchased from Reagent Station of Shenyang Pharmaceutical University) (3 mL) was added to the solution. After stirring overnight at room temperature, the mixture solution was concentrated under reduced pressure and water was added (3 mL), and filtered by suction after adjusting the pH to 8 with saturated sodium bicarbonate solution. The filter cake was further purified by silica-gel column chromatography, to obtain 0.15 g of yellow solid. Yield: 80%.

**Example 9 1-(2-aminobenzo[*d*]thiazol-6-yl)-1-(2-(4-morpholinyl)ethyl)-3-(4-acetylphenyl)urea**

Using 4-acetylaniline (purchased from Beijing Ouhe Technology Co., Ltd.) as raw material, the key Intermediate G was synthesized in accordance with the synthesis process of step 8.2 in Example 8, then Intermediate H was synthesized in accordance with the synthesis process of step 8.3 in Example 8, and then 1-(2-aminobenzo[*d*]thiazol-6-yl)-1-[2-(4-morpholinyl)ethyl]-3-(4-acetylphenyl)urea was synthesized in accordance with the synthesis process of step 8.4 in Example 8. Yield: 54%.

**Example 10 1-(2-aminobenzo[*d*]thiazol-6-yl)-1-[2-(4-morpholinyl)ethyl]-3-(4-acetamidophenyl)urea**

Using 4-acetamidoaniline (purchased from Beijing Ouhe Technology Co., Ltd.) as raw material, the key Intermediate G was synthesized in accordance with the synthesis process of step 8.2 in Example 8, then Intermediate H was synthesized in accordance with the synthesis process of step 8.3 in Example 8, and then 1-(2-aminobenzo[*d*]thiazol-6-yl)-1-[2-(4-morpholinyl)ethyl]-3-(4-acetamidophenyl)urea was synthesized in accordance with the synthesis process of step 8.4 in Example 8. Yield: 69%.

**Example 11 *N*-(4-{3-(2-aminobenzol[*d*]thiazol-6-yl)-3-[2-(4-morpholinyl)ethyl]ureido}phenyl)acetamide**

Using 4-chlorophenylacetic acid (purchased from Beijing Ouhe Technology Co., Ltd.) as raw material, the key Intermediate G was synthesized in accordance with the synthesis process of step 8.2 in Example 8, then Intermediate H was synthesized in accordance with the synthesis process of step 8.3 in Example 8, and then *N*-(4-{3-(2-aminobenzo[*d*]thiazol-6-yl)-3-[2-(4-morpholinyl)ethyl]ureido}phenyl)acetamide was synthesized in accordance with the synthesis process of step 8.4 in Example 8. Yield: 72%.

**Example 12 1-(2-aminobenzo[*d*]thiazol-6-yl)-1-[2-(4-morpholinyl)ethyl]-3-(4-chlorophenyl)urea**

Using 4-chloroaniline (purchased from Beijing Ouhe Technology Co., Ltd.) as raw material, the key Intermediate G was synthesized in accordance with the synthesis process of step 8.2 in Example 8, then Intermediate H was synthesized in accordance with the synthesis process of step 8.3 in Example 8, and then 1-(2-aminobenzo[*d*]thiazol-6-yl)-1-[2-(4-morpholinyl)ethyl]-3-(4-chlorophenyl)urea was synthesized in accordance with the synthesis process of step 8.4 in Example 8. Yield: 57%.

**Example 13 1-(2-aminobenzo[*d*]thiazol-6-yl)-1-[2-(4-morpholinyl)ethyl]-3-(4-trifluoromethylphenyl)urea**

Using 4-trifluoromethylaniline (purchased from Beijing Ouhe Technology Co., Ltd.) as raw material, the key Intermediate G was synthesized in accordance with the synthesis process of step 8.2 in Example 8, then Intermediate H was synthesized in accordance with the synthesis process of step 8.3 in Example 8, and then 1-(2-aminobenzo[*d*]thiazol-6-yl)-1-[2-(4-morpholinyl)ethyl]-3-(4-trifluoromethylphenyl)urea was synthesized in accordance with the synthesis process of step 8.4 in Example 8. Yield: 43%.

**Example 14 1-(2-aminobenzo[*d*]thiazol-6-yl)-1-[2-(4-morpholinyl)ethyl]-3-(pyridin-2-yl)urea**

Using 2-aminopyridine (purchased from Beijing Ouhe Technology Co., Ltd.) as raw material, the key Intermediate G was synthesized in accordance with the synthesis process of step 8.2 in Example 8, then Intermediate H was synthesized in accordance with the synthesis process of step 8.3 in Example 8, and then 1-(2-aminobenzo[*d*]thiazol-6-yl)-1-[2-(4-morpholinyl)ethyl]-3-(pyridin-2-yl)urea was synthesized in accordance with the synthesis process of step 8.4 in Example 8. Yield: 38%.

**Example 15 1-(2-aminobenzo[*d*]thiazol-6-yl)-1-[2-(4-morpholinyl)ethyl]-3-(naphthalen-1-yl)urea**

Using 2-naphthylamine (purchased from Beijing Ouhe Technology Co., Ltd.) as raw material, the key Intermediate G was synthesized in accordance with the synthesis process of step 8.2 in Example 8, then Intermediate H was synthesized in accordance with the synthesis process of step 8.3 in Example 8, and then 1-(2-aminobenzo[*d*]thiazol-6-yl)-1-[2-(4-morpholinyl)ethyl]-3-(naphthalen-1-yl)urea was synthesized in accordance with the synthesis process of step 8.4 in Example 8. Yield: 59%.

**Example 16 1-(2-aminobenzo[*d*]thiazol-6-yl)-1-[2-(4-morpholinyl)ethyl]-3-(3-bromo-4-fluorophenyl)urea**

Using 3-bromo-4-fluoroaniline (purchased from Beijing Ouhe Technology Co., Ltd.) as raw material, the key Intermediate G was synthesized in accordance with the synthesis process of step 8.2 in Example 8, then Intermediate H was synthesized in accordance with the synthesis process of step 8.3 in Example 8, and then 1-(2-aminobenzo[*d*]thiazol-6-yl)-1-[2-(4-morpholinyl)ethyl]-3-(3-bromo-4-fluorophenyl)urea was synthesized in accordance with the synthesis process of step 8.4 in Example 8. Yield: 75%.

**Example 17 1-(2-aminobenzo[*d*]thiazol-6-yl)-1-[2-(4-morpholinyl)ethyl]-3-(4-trifluoromethoxyphenyl)urea**

Using 4-trifluoromethoxyaniline (purchased from Beijing Ouhe Technology Co., Ltd.) as raw material, the key Intermediate G was synthesized in accordance with the synthesis process of step 8.2 in Example 8, then Intermediate H was synthesized in accordance with the synthesis process of step 8.3 in Example 8, and then 1-(2-aminobenzo[*d*]thiazol-6-yl)-1-[2-(4-morpholinyl)ethyl]-3-(4-trifluoromethoxyphenyl)urea was synthesized in accordance with the synthesis process of step 8.4 in Example 8. Yield: 52%.

**Example 18 1-(2-aminobenzo[*d*]thiazol-6-yl)-1-[2-(4-morpholinyl)ethyl]-3-(4-ethylphenyl)urea**

Using p-ethylaniline (purchased from Beijing Ouhe Technology Co., Ltd.) as raw material, the key Intermediate G was synthesized in accordance with the synthesis process of step 8.2 in Example 8, then Intermediate H was synthesized in accordance with the synthesis process of step 8.3 in Example 8, and then 1-(2-aminobenzo[*d*]thiazol-6-yl)-1-[2-(4-morpholinyl)ethyl]-3-(4-ethylphenyl)urea was synthesized in accordance with the synthesis process of step 8.4 in Example 8. Yield: 85%.

**Example 19 1-(2-aminobenzo[*d*]|thiazol-6-yl)-1-[2-(4-morpholinyl)ethyl]-3-(4-isopropoxyphenyl)urea**

Using 4-isopropoxyaniline (purchased from Beijing Ouhe Technology Co., Ltd.) as raw material, the key Intermediate G was synthesized in accordance with the synthesis process of step 8.2 in Example 8, then Intermediate H was synthesized in accordance with the synthesis process of step 8.3 in Example 8, and then 1-(2-aminobenzo[*d*]thiazol-6-yl)-1-[2-(4-morpholinyl)ethyl]-3-(4-isopropoxyphenyl)urea was synthesized in accordance with the synthesis process of step 8.4 in Example 8. Yield: 91%.

**Example 20 1-(2-aminobenzo[*d*]thiazol-6-yl)-1-[2- (4-morpholinyl)ethyl]-3-(4-isopropylphenyl)urea**

Using 4-isopropylaniline (purchased from Beijing Ouhe Technology Co., Ltd.) as raw material, the key Intermediate G was synthesized in accordance with the synthesis process of step 8.2 in Example 8, then Intermediate H was synthesized in accordance with the synthesis process of step 8.3 in Example 8, and then 1-(2-aminobenzo[*d*]thiazol-6-yl)-1-[2-(4-morpholinyl)ethyl]-3-(4-isopropylphenyl)urea was synthesized in accordance with the synthesis process of step 8.4 in Example 8. Yield: 78%.

**Example 21 1-(2-aminobenzo[*d*]thiazol-6-yl)-1-[2-(*N,N*-diethylamino)ethyl]-3-(4-chlorophenyl)urea**

Using 2-chloro-*N,N*-dimethylethyl-1-amine (purchased from Shanghai Bide Pharmatech Ltd.) as raw material, the key Intermediate F was synthesized in accordance with the synthesis process of step 8.1 in Example 8, then using p-chloroaniline as raw material, Intermediate G was synthesized *in* accordance with the synthesis process of step 8.2

in Example 8, and then 1-(2-aminobenzo[*d*]thiazol-6-yl)-1-[2-(*N,N*-diethylamino)ethyl]-3-(4-chlorophenyl)urea was synthesized in accordance with the synthesis process of step 8.4 in Example 8. Yield: 54%.

**Example 22 1-(2-aminobenzo[*d*]thiazol-6-yl)-1-[2-(4-methylpiperidin-1-yl)ethyl)-3-(4-chlorophenyl)urea**

Using 1-(2-chloroethyl)-4-methylpiperidine (purchased from Shanghai Bide Pharmatech Ltd.) as raw material, the key Intermediate F was synthesized in accordance with the synthesis process of step 8.1 in Example 8, then using p-chloroaniline as raw material, Intermediate G was synthesized in accordance with the synthesis process of step 8.2 in Example 8, and then 1-(2-aminobenzo[*d*]thiazol-6-yl)-1-[2-(4-methylpiperidin-1-yl)ethyl]-3-(4-chlorophenyl)urea was synthesized in accordance with the synthesis process of step 8.4 in Example 8. Yield: 65%.

**Example 23 1-(2-aminobenzo[*d*]thiazol-6-yl)-1-[2-(4-methylpiperazin-1-yl)ethyl]-3-(4-trifluoromethylphenyl)urea**

Using 1-(2-chloroethyl)-4-methylpiperazine (purchased from Shanghai Bide Pharmatech Ltd.) as raw material, the key Intermediate F was synthesized in accordance with the synthesis process of step 8.1 in Example 8, then using p-chloroaniline as raw material, Intermediate G was synthesized in accordance with the synthesis process of step 8.2 in Example 8, and then 1-(2-aminobenzo[*d*]thiazol-6-yl)-1-[2-(4-methylpiperazin-1-yl)ethyl]-3-(4-trifluoromethylphenyl)urea was synthesized in accordance with the synthesis process of step 8.4 in Example 8. Yield: 43%.

**Example 24 1-(2-aminobenzo[*d*]thiazol-6-yl)-1-12-(pyrrolidin-1-yl)ethyl]-3-(4-chlorophenyl)urea**

Using 1-(2-chloroethyl)pyrrolidine (purchased from Shanghai Bide Pharmatech Ltd.) as raw material, the key Intermediate F was synthesized in accordance with the synthesis process of step 8.1 in Example 8, then using p-chloroaniline as raw material, Intermediate G was synthesized in accordance with the synthesis process of step 8.2 in Example 8, and then 1-(2-aminobenzo[*d*]thiazol-6-yl)-1-[2-(pyrrolidin-1-yl)ethyl]-3-(4-chlorophenyl)urea was synthesized in accordance with the synthesis process of step 8.4 in Example 8. Yield: 51%.

**Example 25 1-(2-aminobenzo[*d*]thiazol-6-yl)-1-[2-(*N,N*-dimethylamino)ethyl]-3-(4-chlorophenyl)urea**

Using 2-chloro-*N,N*-dimethylethyl-1-amine (purchased from Shanghai Bide Pharmatech Ltd.) as raw material, the key Intermediate F was synthesized in accordance with the synthesis process of step 8.1 in Example 8, then using p-chloroaniline as raw material, Intermediate G was synthesized in accordance with the synthesis process of step 8.2 in Example 8, and then 11-(2-aminobenzo[*d*]thiazol-6-yl)-1-[2-(*N,N*-dimethylamino)ethyl]-3-(4-chlorophenyl)urea was synthesized in accordance with the synthesis process of step 8.4 in Example 8. Yield: 68%.

**Example 26 1-(2-aminobenzo[*d*]thiazol-6-yl)-1-[3-(4-morpholinyl)propyl]-3-(4-chlorophenyl)urea**

Using 4-(3-chloropropyl)morpholine (purchased from Shanghai Bide Pharmatech Ltd.) as raw material, the key Intermediate F was synthesized in accordance with the synthesis process of step 8.1 in Example 8, then using p-chloroaniline as raw material, Intermediate G was synthesized in accordance with the synthesis process of step 8.2 in Example 8, and then 1-(2-aminobenzo[d]thiazol-6-yl)-1-[3-(4-morpholinyl)propyl]-3-(4-chlorophenyl)urea was synthesized in accordance with the synthesis process of step 8.4 in Example 8. Yield: 76%.

**Example 27 1-(2-phenylacetamidobenzo[d]thiazol-6-yl)-1-[2-(4-morpholinyl)ethyl]-3-(4-chlorophenyl)urea**

1-(2-Aminobenzo[*d*]thiazol-6-yl)-3-(4-chlorophenyl)-1-[2-(4-morpholinyl)ethyl]urea (0.2 g, 0.4 mmol) was dissolved in ethyl acetate (3 mL), and potassium carbonate (purchased from Shenyang Aiketongsheng Chemical Reagent Co., Ltd.) (0.1 g, 0.7 mmol) was added. At room temperature, phenylacetic acid (0.1 g, 0.4 mmol) was dropwise added to the solution, and stirred overnight at room temperature. After the reaction was completed, water (5 mL) was added, and extracted with ethyl acetate ($2\times10$ mL). The organic layer was concentrated under reduced pressure, and purified by column chromatography, to obtain 0.08 g of white solid. Yield: 76%.

**Example 28 1-[2-(2-ethoxyformamido)benzo[*d*]thiazol-6-yl]-1-[2-(4-morpholnyl)ethyl]-3-(4-chlorophenyl)urea**

Using ethyl formate (purchased from Beijing Ouhe Technology Co., Ltd.) as raw material, 1-[2-(2-ethoxyformamido)benzo[*d*]thiazol-6-yl]-1-[2-(4-morpholinyl)ethyl]-3-(4-chlorophenyl)urea was prepared in accordance with the synthesis process in Example 27. Yield: 45%.

**Example 29 1-{[2-(2-ethoxyformyl)ethylamino-1-yl]benzo[*d*]thiazol-6-yl}-1-[2-(4-morpholinyl)ethyl]-3-(4-chlorophenyl)urea**

Using ethyl bromoacetate (purchased from Shanghai Bide Pharmatech Ltd.) as raw material, 1-[2-(2-ethoxyformyl)ethylamino-1-yl]benzo[*d*]thiazol-6-yl]-1-[2-(4-morpholinyl)ethyl]-3-(4-chlorophenyl)urea was prepared in accordance with the synthesis process in Example 27. Yield: 32%.

**Example 30 1-(2-acetamidobenzo[*d*]thiazol-6-yl)-1-[2-(4-morpholinyl)ethyl]-3-(4-chlorophenyl)urea**

Using acetyl chloride (purchased from Shanghai Bide Pharmatech Ltd.) as raw material, 1-(2-acetamidobenzo[*d*]thiazol-6-yl)-1-[2-(4-morpholinyl)ethyl]-3-(4-chlorophenyl)urea was prepared in accordance with the synthesis process in Example 27. Yield: 82%.

**Example 31 tert-butyl (6-{2-(4-chlorophenyl)-*N*-[2-(4-morpholinyl)ethyl)acetamido}benzo[*d*]thiazol-2-yl)carbamate**

Using tert-butoxyformyl chloride (purchased from Shanghai Bide Pharmatech Ltd.) as raw material, tert-butyl (6-{2-(4-chlorophenyl)-*N*-[2-(4-morpholinyl)ethyl]acetamido}benzo[*d*]thiazol-2-yl)carbamate was prepared in accordance with the synthesis process in Example 27. Yield: 60%.

**Example 32 1-(2-phenylformamidobenzo[*d*]thiazol-6-yl)-1-[2-(4-morpholinyl)ethyl]-3-(4-chlorophenyl)urea**

Using benzoyl chloride (purchased from Shanghai Bide Pharmatech Ltd.) as raw material, 1-(2-phenylformamidobenzo[*d*]thiazol-6-yl)-1-[2-(4-morpholinyl)ethyl]-3-(4-chlorophenyl)urea was prepared in accordance with the synthesis process in Example 23. Yield: 75%.

**Example 33 1-(2-phenylpropionamidobenzo[*d*]thiazol-6-yl)-1-[2-(4-morpholinyl)ethyl]-3-(4-chlorophenyl)urea**

Using phenylpropionyl chloride (purchased from Shanghai Bide Pharmatech Ltd.) as raw material, 1-(2-phneylpropionamidobenzo[*d*]thiazol-6-yl)-1-[2-(4-morpholinyl)ethyl]-3-(4-chlorophenyl)urea was prepared in accordance with the synthesis process in Example 27. Yield: 68%.

**Example 34 1-(2-phenylacetamidobenzo[*d*]thiazol-6-yl)-1-[2-(4-morpholinyl)ethyl)-3-(4-trifluoromethylphenyl)urea**

Using phenylacetyl chloride (purchased from Shanghai Bide Pharmatech Ltd.) as raw material, 1-(2-phenylacetamidobenzo[*d*]thiazol-6-yl)-1-[2-(4-morpholinyl)ethyl]-3-(4-trifluoromethylphenyl)urea was prepared in accordance with the synthesis process in Example 27. Yield: 73%.

**Example 35 1-(2-aminobenzo[d]oxazol-6-yl)-1-[2-(4-morpholinyl)ethyl]-3-(4-methylphenyl)urea**

**35.1 Synthesis of 6-nitrobenzo[*d*]oxazol-2-amine (K)**

2-Amino-5-nitrophenol (J) (purchased from Shanghai Bide Pharmatech Ltd.) (10 g, 64.9 mmol) was dissolved in 1,4-dioxane (100 mL), and cyanoguanidine (purchased from Beijing Ouhe Technology Co., Ltd.) (17 g, 202.3 mmol) and $BF_3{\bullet}Et_2O$ (purchased from Shanghai Bide Pharmatech Ltd.) (28 g, 202.3 mmol) were successively added thereto. The tube was sealed quickly, and reacted at 80 °C for 6 h. After the reaction solution was cooled to room temperature, 2 N sodium bicarbonate solution was added to the solution, and extracted with ethyl acetate (3×100 mL). The organic layers were combined, washed with brine (100 mL), dried over sodium sulfate, concentrated and dried under reduced pressure, to obtain 8.4 g of Intermediate K as brown solid. Yield: 70%.

**35.2 Synthesis of benzo[*d*]oxazol-2,6-diamine (L)**

Intermediate K (8.0 g, 53.7 mmol) was dissolved in methanol (80 mL), and Pd/C (purchased from Shanghai Bide Pharmatech Ltd.) (0.8 g, 5.4 mmol) was added to the solution. The reaction was maintained by feeding hydrogen at room temperature for 12 h. The mixture solution was filtered through celite, the filter cake was washed with methanol (40 mL), and the filtrate was concentrated under vacuum, to obtain 5.4 g of Intermediate L. Yield: 60%. MS (ESI) m/z: 150.2 $[M+H]^+$.

**35.3 Synthesis of *N*6-[2-(4-morpholinyl)ethyl]benzo[*d*]oxazol-2,6-diamine (M)**

Intermediate L (2.0 g, 13.4 mmol) was dissolved in 1.4-dioxane (20 mL), and potassium carbonate (purchased from Shenyang Aiketongsheng Chemical Reagent Co., Ltd.) (3.1 g, 22.4 mmol) and 4-(2-chloroethyl)morpholine (purchased from Shanghai Bide Pharmatech Ltd.) (1.2 g, 11.2 mmol) were added to the solution. The reaction solution was heated to reflux and reacted under stirring for 8 h. After the reaction was cooled to room temperature, water (80 mL) was added thereto, and extracted with ethyl acetate (2×80 mL). The organic layers were combined, washed with brine (60 mL), dried over sodium sulfate, concentrated and dried under reduced pressure, to obtain 1.5 g of Intermediate M as brown solid. Yield: 72%.

**35.4 Preparation of 1-(2-aminobenzo[*d*]oxazol-6-yl)-1-[2-(4-morpholinyl)ethyl]-3-(4-methylphenyl)urea (I)**

At room temperature, Intermediate M (0.50 g, 0.6 mmol) and phenyl p-toluene methylcarbamate (G) (0.4 g, 0.7 mmol) were added to 1,4-dioxane (4 mL), and DIPEA (purchased from Shanghai Bide Pharmatech Ltd.) (0.3 g, 2.3 mmol) was dropwise added to the reaction solution. After dropwise addition, the system was heated to 60 °C and reacted for 3 h. After the reaction was completed, the solution was cooled to room temperature, and the solvent was concentrated under reduced pressure. To the residue water (10 mL) was added. After adjusting the pH to 10 with 2.5% NaOH solution, the solution was filtered by suction, and dried, to obtain a crude product. The crude product was purified by column chromatography (dichloromethane:methanol=20:1), to obtain 0.35 g of white solid. Yield: 62%.

**Example 36 1-(2-acetamidobenzo[*d*]oxazol-6-yl)-1-[2-(4-morpholinyl)ethyl]-3-(4-methylphenyl)urea**

1-(2-Aminobenzo[*d*]oxazol-6-yl)-1-[2-(4-morpholinyl)ethyl]-3-(4-methylphenyl)urea (0.2 g, 0.4 mmol) was dissolved in ethyl acetate (3 mL), and potassium carbonate (purchased from Shenyang Aiketongsheng Chemical Reagent Co., Ltd.) (0.1 g, 0.7 mmol) was added. At room temperature, acetyl chloride (0.1 g, 0.4 mmol) was dropwise added to the solution, and stirred overnight at room temperature. After the reaction was completed, water (5 mL) was added, and extracted with ethyl acetate (2×10 mL). The organic layer was concentrated under reduced pressure, and purified by column chromatography, to obtain 0.08 g of white solid. Yield: 76%.

**Example 37 1-(2-phenylacetamidobenzo[*d*]oxazol-6-yl)-1-[2-(4-morpholinyl)ethyl]-3-(4-methylphenyl)urea**

Using *p*-chlorophenylacetyl chloride (purchased from Shanghai Bide Pharmatech Ltd.) as raw material, 1-(2-phenylacetamidobenzo[*d*]oxazol-6-yl)-1-[2-(4-morpholinyl)ethyl]-3-(4-methylphenyl)urea was prepared in accordance with the synthesis process in Example 36. Yield: 60%.

**Example 38 1-(2-aminothiazo[5,4-*b*]pyridin-5-yl)-3-(4-fluorophenyl)-1-[2-(4-morpholinyl)ethyl]urea**

**38.1 Synthesis of 5-chlorothiazo[5,4-*b*]pyridin-2-amine (O)**

2-Chloro-S-aminopyridine (N) (10 g, 78.1 mmol) and potassium thiocyanate (purchased from Shanghai Bide Pharmatech Ltd.) (15 g, 312.4 mmol) were dissolved in glacial acetic acid (100 mL), and bromine (purchased from Reagent Station of Shenyang Pharmaceutical University) (12 g, 156.2 mmol) was dropwise added thereto in ice bath. After dropwise addition, the reaction solution was heated to room temperature and reacted for 12 h. After adjusting the pH to 10 by adding ammonia water into the solution, a yellow solid was precipitated and filtered by suction, to obtain 12.2 g of Intermediate 0 as brown solid. Yield: 83%.

**38.2 Synthesis of *N*5-[2-(4-morpholinyl)ethy[*d*]thiazo[5,4-*b*]pyridin-2,5-diamine (P)**

Intermediate O (2.0 g, 13.4 mmol) was dissolved in 1.4-dioxane (20 mL), and sodium hydroxide (purchased from Shenyang Aiketongsheng Chemical Reagent Co., Ltd.) (2.5 g, 22.4 mmol) and 2-morpholino-1-amine (purchased from Shanghai Bide Pharmatech Ltd.) (1.2 g, 11.2 mmol) were added to the solution. The reaction solution was heated to reflux and reacted under stirring for 8 h. After the reaction was cooled to room temperature, water (30 mL) was added thereto, and extracted with ethyl acetate (2×30 mL). The organic layers were combined, washed with brine (60 mL), dried over sodium sulfate, concentrated and dried under reduced pressure, to obtain 1.3 g of Intermediate P as brown solid. Yield: 54%.

**38.3 Preparation of 1-(2-aminothiazo[5,4-*b*]pyridin-5-yl)-3-(4-fluorophenyl)-1-[2-(4-morpholinyl)ethyl)urea (I)**

At room temperature, Intermediate P (0.5 g, 0.6 mmol) and phenyl p-fluorobenzylcarbamate (G) (0.4 g, 0.7 mmol) were added to 1,4-dioxane (4 mL), and DIPEA (purchased from Shanghai Bide Pharmatech Ltd.) (0.30 g, 2.3 mmol) was dropwise added to the reaction solution. After dropwise addition, the system was heated to 60 °C and reacted for 3 h. After the reaction was completed, the solution was cooled to room temperature, and the solvent was concentrated under reduced pressure. To the residue water (10 mL) was added. After adjusting the pH to 10 with 2.5% NaOH solution, the solution was filtered by suction, and dried, to obtain a crude product. The crude product was purified by column chromatography (dichloromethane:methanol=20:1), to obtain 0.3 g of white solid. Yield: 51%.

**Example 39 1-[2-(ethylamino)thiazo[5,4-*b*]pyridin-5-yl]-1-[2-(4-morpholinyl)ethyl]-3-(4-fluorophenyl)urea**

1-(2-Aminothiazo[5,4-*b*]pyridin-5-yl)-3-(4-fluorophenyl)-1-[2-(4-morpholinyl)ethyl]urea (0.2 g, 0.4 mmol) was dissolved in ethyl acetate (3 mL), and potassium carbonate (purchased from Shenyang Aiketongsheng Chemical Reagent Co., Ltd.) (0.1 g, 0.7 mmol) was added to the solution, and stirred overnight at room temperature. After the reaction was completed, water (10 mL) was added, and extracted with ethyl acetate (2×30 mL). The organic layer was concentrated under reduced pressure, and purified by column chromatography, to obtain 0.08 g of white solid. Yield: 65%.

**Example 40 1- {2-[3-(4-chlorophenyl)ureido]benzo[*d*]thiazol-6-yl}-1-[2-(4-morpholinyl)ethyl]-3-(4-chlorophenyl)urea**

At room temperature, 1-(2-aminobenzo[*d*]thiazol-6-yl)-3-(4-chlorophenyl)-1-[2-(4-morpholinyl)ethyl]urea (0.5 g, 0.6 mmol) and phenyl benzylaminocarbamate (purchased from Shanghai Bide Pharmatech Ltd.) (0.4 g, 0.7 mmol) were added to 1,4-dioxane (4 mL), and triethylamine0.3 g(2.3 mmol) was dropwise added to the reaction solution. After dropwise addition, the system was heated to 60 °C and reacted for 3 h. After the reaction was completed, the solution was cooled to room temperature, and the solvent was concentrated under reduced pressure. To the residue water (10 mL) was added. After adjusting the pH to 10 with 2.5% NaOH solution, the solution was filtered by suction, and dried, to obtain a crude product. The crude product was purified by column chromatography (dichloromethane:methanol=20:1), to obtain 0.25 g of white solid. Yield: 40%.

**Example 41 1-(2-aminothiazo[4,5-*c*]pyridin-6-yl)-1-[2-(4-morpholinyl)ethyl]-3-(4-trifluoromethoxyphenyl)urea**

**41.1 Synthesis of *N*-[(4,6-dichloropyridin-3-yl)thiocarbamoyl]benzamide (R)**

4,6-Dichloropyridin-3-amine (Q) (5 g, 30.7 mmol) and phenyl isothiocyanate (purchased from Shanghai Bide Pharmatech Ltd.) (4.5 mL, 33.7 mmol) were dissolved in acetone (75 mL), heated to 60 °C, and reacted for 4 h. The reaction solution was concentrated under reduced pressure, to obtain 8 g of brown solid. Yield: 80%.

**41.2 Synthesis of *N*-(6-chlorothiazo[4,5-*c*]pyridin-2-yl)benzamide (S)**

Intermediate R (9 g, 27.7 mmol) was added to NMP (45 mL), and at 0 ° C, sodium methoxide (purchased from Shanghai Bide Pharmatech Ltd.) (3.0 g, 55.8 mmol) was added to the solution. After addition, the reaction solution was heated to 120 °C and reacted for 4 h. Water (100 mL) was added to the reaction solution, and a brown solid was precipitated, which was filtered by suction to obtain 7 g of brown solid. Yield: 87%.

**41.3 Synthesis of 6-chlorothiazo[4,5-*c*]pyridin-2-amine (T)**

Intermediate S (5 g, 17.3 mmol) was dissolved in 70% sulfuric acid solution (purchased from Reagent Station of Shenyang Pharmaceutical University) (15 mL), and heated to 110 °C and reacted for 4 h. The reaction solution was concentrated under reduced pressure, adjusted to pH = 8 with sodium bicarbonate solution (20 mL), and filtered by suction to obtain 1.8 g of offwhite solid. Yield: 56%.

**41.4 Synthesis of $N^6$-[2-(4-morpholinyi)ethylithiazo[4,5-*c*]pyridin-2,6-diamine (U)**

Intermediate T (0.8 g, 25.1 mmol) and cesium carbonate (purchased from Shanghai Bide Pharmatech Ltd.) (2.2 g, 75.3 mmol) were dissolved in 1,4-dioxane (10 mL), and palladium acetate (purchased from Shanghai Bide Pharmatech Ltd.) (0.1 g, 2.51 mmol) and xantphos (purchased from Shanghai Bide Pharmatech Ltd.) (0.35 g, 50.2 mmol) were added to the solution. With feeding nitrogen gas, the reaction solution was heated to 100 ° C and reacted

for 12 h. After removing the insoluble matters by suction filtration, the filtrate was purified by column chromatography, to obtain 0.5 g of white solid. Yield: 66%.

**41.5 Preparation of 1-(2-aminothiazo[4,5-*c*]pyridin-6-yl)-1-[2-(4-morpholinyl)ethyl]-3-(4-trifluoromethoxyphenyl)urea (I)**

At room temperature, Intermediate U (0.50 g, 0.6 mmol) and phenyl p-trifluoromethoxybenzylcarbamate (G) (0.4 g, 0.7 mmol) were added to 1,4-dioxane (4 mL), and DIPEA (purchased from Shanghai Bide Pharmatech Ltd.) (0.3 g, 2.3 mmol) was dropwise added to the reaction solution. The system was heated to 60 °C and reacted for 3 h. After the reaction was completed, the solution was cooled to room temperature, and the solvent was concentrated under reduced pressure. To the residue water (10 mL) was added. After adjusting the pH to 10 with 2.5% NaOH solution, the solution was filtered by suction, and dried, to obtain a crude product. The crude product was purified by column chromatography (dichloromethane:methanol=20:1), to obtain 0.35 g of white solid. Yield: 60%.

**Example 42 1-(2-cyclopropylformamidothiazo[4,5-*c*]pyridin-6-yl)-1-[2-(4-morpholinyl)ethyl]-3-(4- trifluoromethoxyphenyl)u rea**

1-(2-Aminothiazo[4,5-c]pyridin-6-yl)-1-[2-(4-morpholinyl)ethyl]-3-(4-trifluoromethoxyphenyl)urea (0.2 g, 0.4 mmol) was dissolved in ethyl acetate (3 mL), and potassium carbonate (purchased from Shenyang Aiketongsheng Chemical Reagent Co., Ltd.) (0.1 g, 0.7 mmol) was added. At room temperature, cyclopropionyl chloride (0.15 g, 0.4 mmol) was dropwise added to the solution, and stirred overnight at room temperature. After the reaction was completed, water (10 mL) was added, and extracted with ethyl acetate (2×30 mL). The organic layer was concentrated under reduced pressure, and purified by column chromatography, to obtain 0.06 g of white solid. Yield: 62%.

**Example 43 1-{[2-(2-ethoxyformyl)ethylamino-1-yl]thiazo[4,5-*c*]pyridin-6-yl}-1-(2-(4-morpholinyl)ethyl]-3-(4-trifluoromethoxyphenyl)urea**

Using ethyl chloroacetate (purchased from Shanghai Bide Pharmatech Ltd.) as raw material, 1-{[2-(2-ethoxyformyl)ethylamino-1-yl]thiazo[4,5-*c*]pyridin-6-yl}-1-[2-(4-morpholinyl)ethyl]-3-(4-trifluoromethoxyphenyl)urea was prepared in accordance with the synthesis process in Example 42. Yield: 42%.

**Example 44 1-(2-aminothiazo[5,4-*d*]pyrimidin-5-yl)-1-[2-(4-morpholinyl)ethyl]-3-(pyridin-3-yl)urea**

Using 2,4-dichloropyrimidin-5-amine (purchased from Shanghai Bide Pharmatech Ltd.) as raw material, the key Intermediate U was synthesized in accordance with the synthesis processes of steps 41.1 to 41.4 in Example 41, and then 1-(2-aminothiazo[5,4-*d*]pyrimidin-5-yl)-1-[2-(4-morpholinyl)ethyl]-3-(pyridin-3-yl)urea was synthesized in accordance with the synthesis process of step 41.5 in Example 41. Yield: 59%.

**Example 45 1-(2-acetamidothiazo[5,4-*d*]pyrimidin-5-yl)-1-[2-(4-morpholinyl)ethyl]-3-(pyridin-3-yl)urea**

Using acetyl chloride (purchased from Shanghai Bide Pharmatech Ltd.) as raw material, 1-(2-acetamidothiazo[5,4-*d*]pyrimidin-5-yl)-1-[2-(4-morpholinyl)ethyl]-3-(pyridin-3-yl)urea was prepared in accordance with the synthesis process in Example 42. Yield: 42%.

**Example 46 1-(2-phenylpropionamidothiazo[5,4-*d*]pyrimidin-5-yl)-1-[2-(4-morpholinyl)ethyl)-3-(pyridin-3-yl)urea**

Using phenylpropionyl chloride (purchased from Shanghai Bide Pharmatech Ltd.) as raw material, 1-(2-phenylpropionamidothiazo[5,4-*d*]pyrimidin-5-yl)-1-[2-(4-morpholinyl)ethyl]-3-(pyridin-3-yl)urea was prepared in accordance with the synthesis process in Example 42. Yield: 56%.

**Example 47 1-[2-(3-benzylureido)thiazo[4,5-*b*]pyridin-6-yl]-1-[2-(4-morpholinyl)ethyl]-3-(4-bromo-3-fluorophenyl)urea**

Using 3,5-dichloropyridin-2-amine (purchased from Shanghai Bide Pharmatech Ltd.) as raw material, the key Intermediate U was synthesized in accordance with the synthesis processes of steps 41.1 to 41.4 in Example 41, and then 1-(2-aminothiazo[4,5-b]pyridin-6-yl)-3-(4-bromo-3-fluorophenyl)-1-[2-(4-morpholinyl)ethyl]urea was synthesized in accordance with the synthesis process of step 41.5 in Example 41. Then 1-(2-aminothiazo[4,5-*b*]pyridin-6-yl)-3-(4-bromo-3-fluorophenyl)-1-[2-(4-morpholinyl)ethyl]urea and phenyl benzylcarbamate (0.4 g, 0.7 mmol) were added to 1,4-dioxane (4 mL), and triethylamine (0.3 g, 2.3 mmol) was dropwise added to the reaction solution. The system was heated to 60 °C and reacted for 3 h. After the reaction was completed, the solution was cooled to room temperature, and the solvent was concentrated under reduced pressure. To the residue water (10 mL) was added. After adjusting the pH to 10 with 2.5% NaOH solution, the solution was filtered by suction, and dried, to obtain a crude product. The crude product was purified by column chromatography (dichloromethane:methanol=20:1), to obtain 0.25 g of white solid. Yield: 40%.

The structures as well as $^1$H-NMR and MS data for the compounds of Examples are shown in Table 1.

**Table 1**

| Example | Structural Formula | $^1$H-NMR, MS m/z data |
|---|---|---|
| **1** | 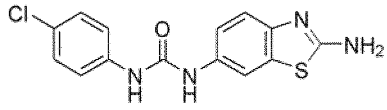 | MS (ESI) m/z: 319.6 [M+H]$^+$ |
| **2** | 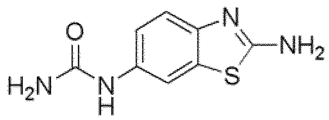 | $^1$H NMR (400 MHz, DMSO-$d_6$) δ 8.41 (s, 1H), 7.83 (d, *J* = 1.9 Hz, 1H), 7.24 (s, 2H), 7.19 (d, *J* = 8.6 Hz, 1H), 7.06 (dd, *J* = 8.6, 2.0 Hz, 1H), 5.78 (s, 2H).MS (ESI) m/z: 209.5 [M+H]$^+$ |
| **3** | 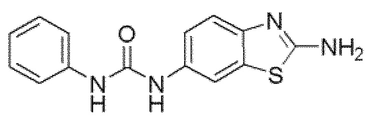 | $^1$H NMR (400 MHz, DMSO-$d_6$) δ 9.04 (s, 1H), 8.99 (s, 1H), 7.88 (d, *J* = 1.6 Hz, 1H), 7.48 (d, *J* = 7.9 Hz, 2H), 7.29 (d, *J* = 1.9 Hz, 2H), 7.26 (s, 1H), 7.24 (d, *J* = 3.6 Hz, 1H), 7.19 (dd, *J* = 8.6, 1.7 Hz, 1H), 6.94 (t, *J* = 7.3 Hz, 1H). MS (ESI) m/z: 285.3 [M+H]$^+$ |
| **4** | 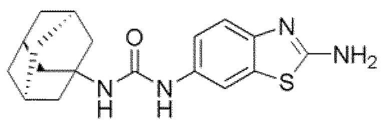 | $^1$H NMR (400 MHz, DMSO-$d_6$)δ 8.45 (s, 1H), 7.82 (d, *J* = 2.1 Hz, 1H), 7.23 (s, 2H), 7.18 (d, *J* = 8.6 Hz, 1H), 7.06 (dd, *J* = 8.6, 2.2 Hz, 1H), 2.09 (s, 1H), 1.97 (s, 2H), 1.62 (d, *J* = 14.8 Hz, 2H), 1.57 (s, 3H), 1.53 (s, 2H), 1.50 (s, 1H), 1.48 (d, *J* = 2.3 Hz, 4H).MS (ESI) m/z: 343.4 [M+H]$^+$ |
| **5** | 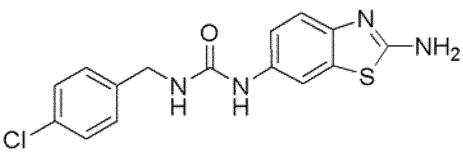 | $^1$H NMR (400 MHz, DMSO-$d_6$) δ 8.69 (s, 1H), 7.83 (s, 1H), 7.38 (d, *J* = 8.4 Hz, 2H), 7.32 (d, *J* = 8.4 Hz, 2H), 7.24 (s, 2H), 7.19 (d, *J* = 8.6 Hz, 1H), 7.10 (d, *J* = 8.6 Hz, l H), 6.83 (s, 1H), 4.27 (d, *J* = 5.8 Hz, 2H).MS (ESI) m/z: 333.5 [M+H]$^+$ |
| **6** | 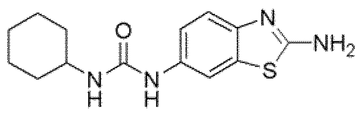 | MS (ESI) m/z: 291.5 [M+H]$^+$ |
| **7** | 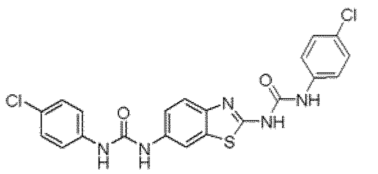 | MS (ESI) m/z: 472.1 [M+H]$^+$ |
| **8** | 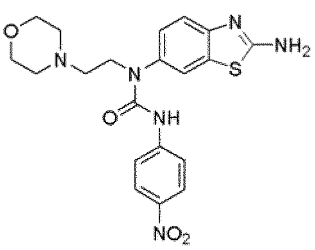 | $^1$H NMR (600 MHz, DMSO-$d_6$) δ 9.66 (s, 2H), 8.19 (d, *J* = 9.3 Hz, 2H), 7.91 (d, *J* = 2.1 Hz, 1H), 7.72 (d, *J* = 9.2 Hz, 2H), 7.31 (d, *J* = 8.6 Hz, 1H), 7.24 (dd, *J* = 8.6, 2.1 Hz, 1H), 3.61 - 3.54 (m 4H), 3.47 (t, *J* = 6.6 Hz, 2H), 2.53 (t, *J* = 6.6 Hz, 2H), 2.42 (s, 4H). MS (ESI) m/z: 443.3 [M+H]$^+$ |

(continued)

| Example | Structural Formula | $^1$H-NMR, MS m/z data |
|---|---|---|
| **9** | 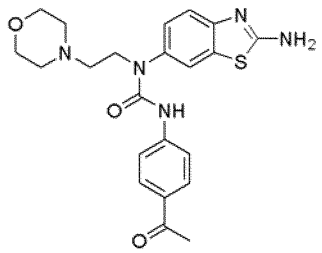 | $^1$H NMR (600 MHz, DMSO-$d_6$) δ 10.25 (s, 1H), 9.90 (s, 1H), 7.90 (s, 2H), 7.89 (s, 2H), 7.62 (s, 1H), 7.60 (s, 1H), 7.29 (d, *J* = 8.6 Hz, 1H), 7.22 (dd, *J* = 8.6, 2.0 Hz, 1 H), 3.58 (t, *J* = 4.5 Hz, 4H), 3.47 (dd, *J* = 12.2, 6.4 Hz, 2H), 2.53 (d, *J* = 6.6 Hz, 2H), 2.51 (s, 3H), 2.42 (s, 4H).MS (ESI) m/z: 440.2[M+H]$^+$ |
| **10** | 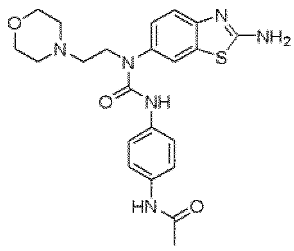 | $^1$H NMR (600 MHz, DMSO-$d_6$) δ 9.77 (s, 1H), 9.30 (d, *J* = 5.4 Hz, 2H), 7.83 (d, *J* = 2.1 Hz, 1H), 7.72 (t, *J* = 5.4 Hz, 1H), 7.39 (d, *J* = 8.9 Hz, 2H), 7.32 (d, *J* = 8.9 Hz, 2H), 7.20 (d, *J* = 8.6 Hz, 1H), 7.13 (dd, *J* = 8.6, 2.1 Hz, 1H), 3.51 (t, *J* = 4.5 Hz, 4H), 3.39 (dd, *J* = 12.2, 6.4 Hz, 2H), 2.47 - 2.44 (m, 2H), 2.35 (s, 4H), 1.94 (s, 3H). MS (ESI) m/z: 455.3[M+H]$^+$ |
| **11** | 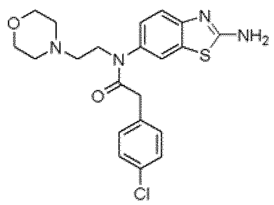 | MS (ESI) m/z: 503.1[M+H]$^+$ |
| **12** | 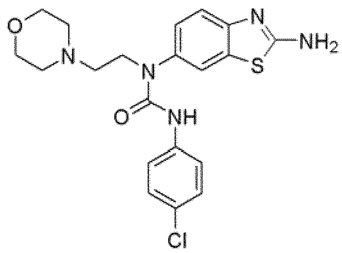 | $^1$H NMR (600 MHz, DMSO-$d_6$) δ 9.62 (s, 1H), 9.53 (s, 1H), 7.99 (s, 1H), 7.50 (d, *J* = 8.4 Hz, 2H), 7.44 (d, *J* = 8.4 Hz, 2H), 7.39 (d, *J* = 8.5 Hz, 1H), 7.30 (d, *J* = 8.3 Hz, 1H), 3.86 (s, 4H), 3.80 (s, 2H), 3.42 (d, *J* = 5.2 Hz, 2H), 3.38 (s, 4H). MS (ESI) m/z: 432.3[M+H]$^+$ |
| **13** | 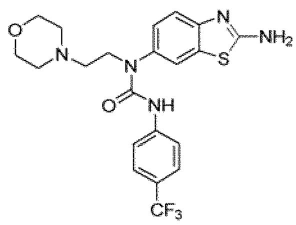 | $^1$H NMR (600 MHz, DMSO-$d_6$) δ 7.63 (s, 1H), 7.37 (d, *J* = 8.6 Hz, 2H), 7.12 (d, *J* = 8.8 Hz, 2H), 6.90 (d, *J* = 8.2 Hz, 1H), 6.63 (d, *J* = 5.0 Hz, 1H), 3.59 (t, *J* = 4.6 Hz, 4H), 2.53 - 2.52 (m, 2H), 2.47 - 2.44 (m, 2H), 2.39 (s, 4H). MS (ESI) m/z: 466.3[M+H]$^+$ |
| **14** | 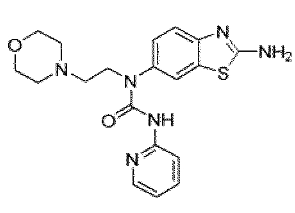 | $^1$H NMR (600 MHz, DMSO-$d_6$) δ 10.58 (s, 1H), 9.45 (s, 1H), 8.28 (dd, *J* = 5.0, 1.1 Hz, 1H), 7.95 (d, *J* = 2.1 Hz, 1H), 7.74 (ddd, *J* = 8.9, 7.4, 1.9 Hz, 1H), 7.45 (d, *J* = 8.4 Hz, 1H), 7.31 (d, *J* = 8.6 Hz, 1H), 7.25 (dd, .7 = 8.6, 2.2 Hz, 1H), 7.02 - 6.98 (m, 1H), 3.58 (t, *J* = 4.6 Hz, 4H), 3.47 (t, *J* = 6.5 Hz, 2H), 2.53 (t, *J* = 6.6 Hz, 2H), 2.42 (s, 4H). MS (ESI) m/z: 399.6[M+H]$^+$ |

(continued)

| Example | Structural Formula | $^1$H-NMR, MS m/z data |
|---|---|---|
| **15** | 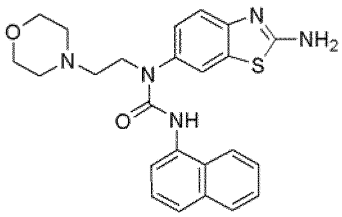 | $^1$H NMR (600 MHz, DMSO-$d_6$) δ 8.25 (dd, *J* = 5.7, 3.8 Hz, 1H), 8.00 (d, *J* = 8.6 Hz, 2H), 7.88 - 7.84 (m, 1H), 7.52 (d, *J* = 7.7 Hz, 2H), 7.50 - 7.47 (m, 1H), 7.42 (d, *J* = 7.8 Hz, 1H), 7.26 (d, *J* = 8.7 Hz, 2H), 3.60 - 3.57 (m, 4H), 3.46 (t, *J* = 6.6 Hz, 4H), 2.53 (d, *J* = 6.6 Hz, 2H), 2.42 (s, 2H). MS (ESI) m/z: 448.3 [M+H]$^+$ |
| **16** | 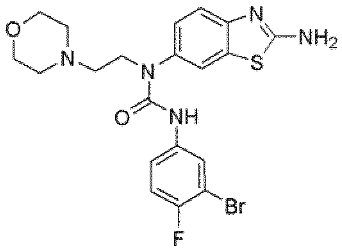 | $^1$H NMR (400 MHz, DMSO-$d_6$) δ 9.23 (s, 2H), 7.95 (dd, *J* = 6.3, 2.6 Hz, 1H), 7.88 (d, *J* = 2.1 Hz, 1H), 7.37 (dd, *J* = 8.9, 4.2, 2.7 Hz, 1H), 7.28 (dt, *J* = 8.8, 4.5 Hz, 2H), 7.19 (dd, *J* = 8.6, 2.1 Hz, 1H), 3.60 - 3.56 (m, 4H), 3.46 (t, *J* = 6.6 Hz, 2H), 2.53 (d, *J* = 6.6 Hz, 2H), 2.42 (s, 4H). MS (ESI) m/z: 494.5[M+H]$^+$ |
| **17** | 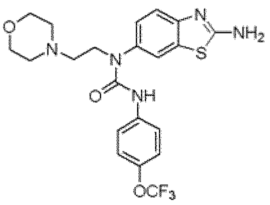 | $^1$H NMR (600 MHz, DMSO-$d_6$) δ 9.48 (s, 1H), 9.21 (s, 1H), 7.87 (d, *J* = 40.0 Hz, 2H), 7.57 (s, 1H), 7.28 (s, 4H), 3.55 (s, 6H), 2.41 (s, 6H).MS (ESI) m/z: 482.6 [M+H]$^+$ |
| **18** | 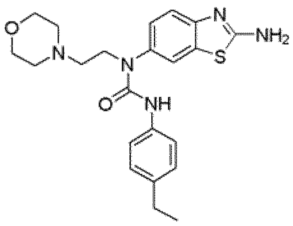 | $^1$H NMR (600 MHz, DMSO-$d_6$) δ 8.59 (s, 2H), 7.88 (d, *J* = 2.0 Hz, 1H), 7.79 (s, 1H), 7.36 (d, *J* = 8.4 Hz, 2H), 7.29 (d, *J* = 8.6 Hz, 1H), 7.16 (dd, *J* = 8.6, 2.1 Hz, 1H), 7.10 (d, *J* = 8.4 Hz, 2H), 3.58 (t, *J* = 4.4 Hz, 4H), 3.48 - 3.45 (m, 2H), 2.54 (t, *J* = 6.2 Hz, 2H), 2.51 (dd, *J* = 5.9, 2.2 Hz, 2H), 2.42 (s, 4H), 1.15 (t, *J* = 7.6 Hz, 3H). MS (ESI) m/z: 426.3 [M+H]$^+$ |
| **19** | 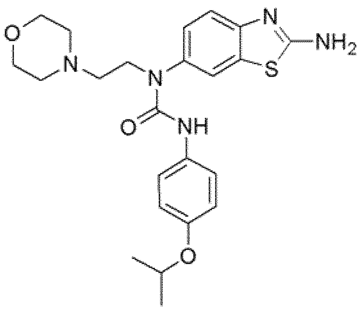 | $^1$H NMR (600 MHz, DMSO-$d_6$) δ 8.97 (s, 1H), 8.90 (s, 1H), 7.88 (s, 1H), 7.77 (s, 1H), 7.35 (d, *J* = 8.6 Hz, 2H), 7.27 (d, *J* = 8.5 Hz, 1H), 7.18 (d, *J* = 8.5 Hz, 1H), 6.83 (d, *J* = 8.6 Hz, 2H), 4.53 - 4.47 (m, 1H), 3.58 (s, 4H), 3.46 (d, *J* = 5.1 Hz, 2H), 3.33 (s, 2H), 2.42 (s, 4H), 1.24 (s, 3H), 1.23 (s, 3H). MS (ESI) m/z: 456.1 [M+H]$^+$ |
| **20** | 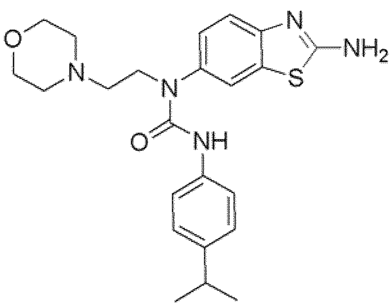 | $^1$H NMR (600 MHz, DMSO-$d_6$) δ 7.89 (d, *J* = 1.8 Hz, 1H), 7.39 (d, *J* = 8.4 Hz, 2H), 7.25 (d, *J* = 8.6 Hz, 1H), 7.19 (dd, *J* = 8.6, 1.7 Hz, 1H), 7.12 (d, *J* = 8.5 Hz, 2H), 3.59 - 3.57 (m, 4H), 3.45 (t, *J* = 6.6 Hz, 2H), 2.85 - 2.79 (m, 1H), 2.53 (d, *J* = 6.7 Hz, 2H), 2.42 (s, 4H), 1.19 (s, 3H), 1.17 (s, 3H). MS (ESI) m/z: 440.2[M+H]$^+$ |

(continued)

| Example | Structural Formula | $^1$H-NMR, MS m/z data |
|---|---|---|
| **21** | 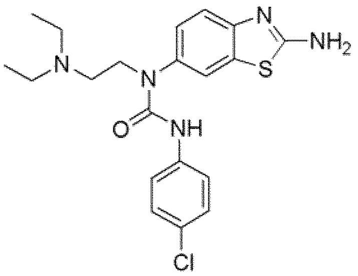 | $^1$H NMR (600 MHz, DMSO-$d_6$) δ 8.82 (s, 1H), 8.64 (s, 1H), 7.85 (d, *J* = 2.2 Hz, 1H), 7.76 (t, *J* = 5.5 Hz, 1H), 7.48 (d, *J* = 8.9 Hz, 2H), 7.31 (d, *J* = 8.9 Hz, 2H), 7.28 (d, *J* = 8.6 Hz, 1H), 7.16 (dd, *J* = 8.6, 2.2 Hz, 1H), 3.39 (dd, *J* = 12.8, 6.3 Hz, 2H), 2.61 (t, *J* = 6.8 Hz, 2H), 2.53 (d, *J* = 7.1 Hz, 2H), 2.50 - 2.49 (m, 2H), 0.97 (t, *J* = 7.1 Hz, 6H).MS (ESI) m/z: 418.5[M+H]$^+$ |
| **22** | 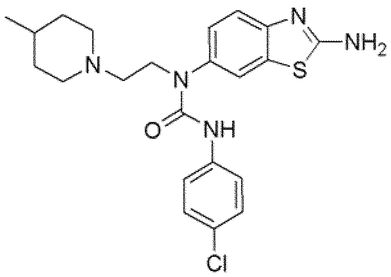 | $^1$H NMR (600 MHz, DMSO-$d_6$) δ 9.76 (s, 1H), 9.58 (s, 1H), 7.89 (s, 1H), 7.75 (s, 1H), 7.54 (d, *J* = 8.7 Hz, 2H), 7.29 (d, *J* = 8.7 Hz, 2H), 7.26 (s, 1H), 7.23 (d, *J* = 9.8 Hz, 1H), 3.43 (d, *J* = 4.9 Hz, 2H), 3.34 (s, 4H), 2.82 (dd, *J* = 22.5, 11.2 Hz, 4H), 1.93 (t, *J* = 10.7 Hz, 3H), 0.87 (t, *J* = 6.7 Hz, 3H). MS (ESI) m/z: 444.3 [M+H]$^+$ |
| **23** | 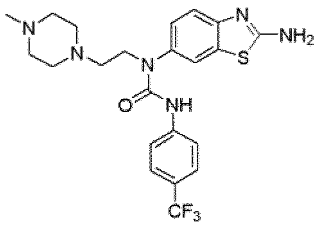 | $^1$H NMR (400 MHz, DMSO-$d_6$) δ 7.93 (s, 1H), 7.67 (d, *J* = 8.5 Hz, 2H), 7.55 (d, *J* = 8.6 Hz, 2H), 7.33 - 7.19 (m, 2H), 3.44 (t, *J* = 6.5 Hz, 2H), 2.51 (s, 4H), 2.43 (s, 2H), 2.32 (d, *J* = 5.2 Hz, 4H), 2.14 (s, 3H), MS (ESI) m/z: 479.2[M+H]$^+$ |
| **24** | 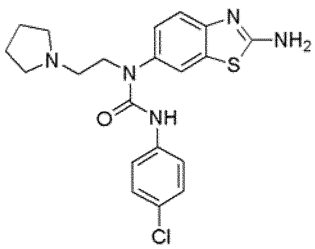 | $^1$H NMR (400 MHz, DMSO-$d_6$) δ 9.57 (s, 1H), 9.39 (s, 1H), 7.88 (s, 1H), 7.83 (d, *J* = 5.3 Hz, 1H), 7.53 (d, *J* = 8.6 Hz, 2H), 7.30 (d, *J* = 8.6 Hz, 2H), 7.22 (d, *J* = 8.5 Hz, 1H), 3.45 (d, *J* = 5.2 Hz, 2H), 2.63 (t, *J* = 6.6 Hz, 2H), 1.69 (s, 4H), 1.23 (s, 4H). MS (ESI) m/z: 416.5 [M+H]$^+$ |
| **25** | 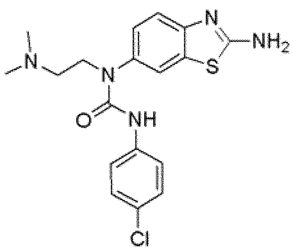 | $^1$H NMR (400 MHz, DMSO-$d_6$) δ 9.12 (s, 1H), 8.95 (s, 1H), 7.80 (d, *J* = 1.9 Hz, 1H), 7.73 (t, *J* = 5.2 Hz, 1H), 7.44 (d, *J* = 8.8 Hz, 2H), 7.25 (d, *J* = 8.8 Hz, 2H), 7.12 (dd, *J* = 8.4, 1.9 Hz, 1H), 2.39 (t, *J* = 6.5 Hz, 2H), 2.12 (s, 6H), 2.11 (s, 2H).MS (ESI) m/z: 390.6[M+H]$^+$ |
| **26** | 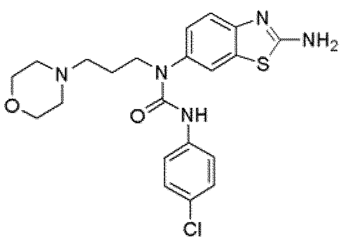 | $^1$H NMR (600 MHz, DMSO-$d_6$) δ 7.85 (s, 1H), 7.47 (d, *J* = 8.6 Hz, 2H), 7.22 (d, *J* = 8.6 Hz, 2H), 7.17 (d, *J* = 8.5 Hz, 1H), 7.13 (d, *J* = 8.3 Hz, 1H), 3.56 (s, 4H), 3.32 (t, *J* = 6.8 Hz, 2H), 2.35 (d, *J* = 7.0 Hz, 2H), 2.33 (s, 4H), 1.75 - 1.71 (m, 2H). MS (ESI) m/z: 446.3[M+H]' |

(continued)

| Example | Structural Formula | $^1$H-NMR, MS m/z data |
|---|---|---|
| **27** | 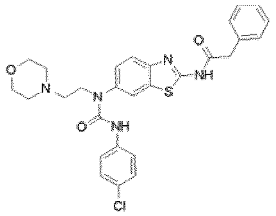 | MS (ESI) m/z:550.6[M+H]$^+$ |
| **28** | 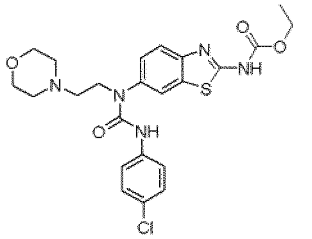 | MS (ESI) m/z:504.5[M+H]$^+$ |
| **29** | 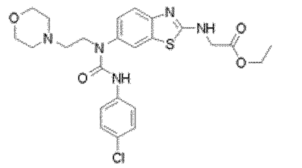 | MS (ESI) m/z:518.3[M+H]$^4$ |
| **30** | 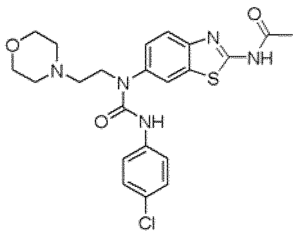 | MS (ESI) m/z:474.1[M+H]$^+$ |
| **31** | 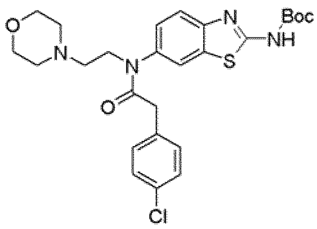 | MS (ESI) m/z:531.5[M+H]$^+$ |
| **32** | 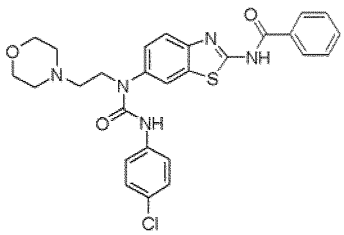 | MS (ESI) m/z:536.4[M+H]$^+$ |
| **33** | 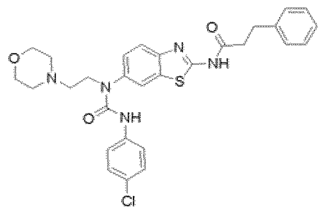 | MS (ESI) m/z:564.2[M+H]$^+$ |

(continued)

| Example | Structural Formula | $^1$H-NMR, MS m/z data |
|---|---|---|
| 34 | 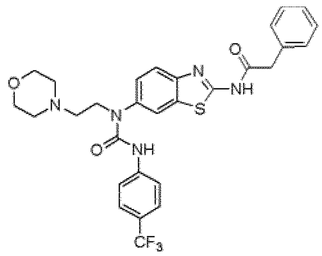 | MS (ESI) m/z:584.3[M+H]$^+$ |
| 35 | 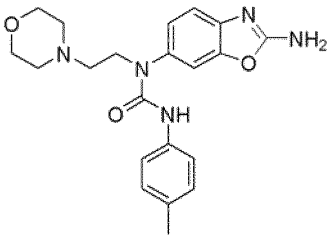 | MS (ESI) m/z:396.2[M+H]$^+$ |
| 36 | 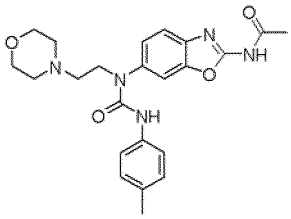 | MS (ESI) m/z:438.5[M+H]$^+$ |
| 37 | 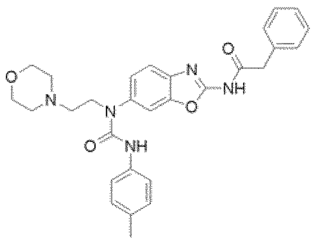 | MS (ESI) m/z:514.1[M+H]$^+$ |
| 38 | 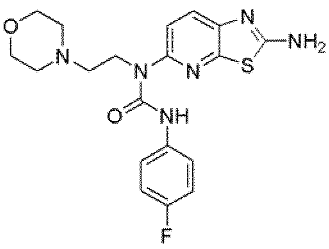 | MS (ESI) m/z:417.5[M+H]$^+$ |
| 39 | 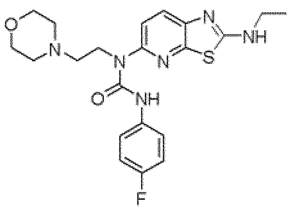 | MS (ESI) m/z:445.3[M+H]$^+$ |

(continued)

| Example | Structural Formula | $^1$H-NMR, MS m/z data |
|---|---|---|
| **40** | 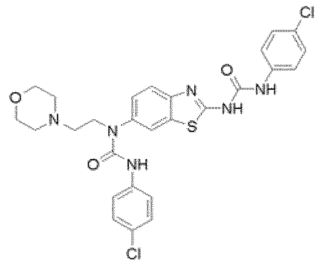 | MS (ESI) m/z:585.1[M+H]$^+$ |
| **41** | 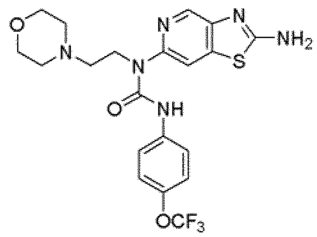 | MS (ESI) m/z:483.4[M+H]$^+$ |
| **42** | 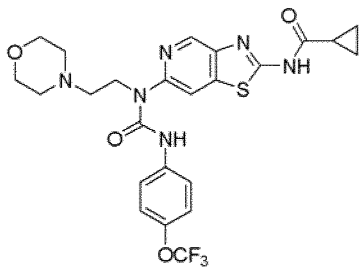 | MS (ESI) m/z:551.1[M+H]$^+$ |
| **43** | 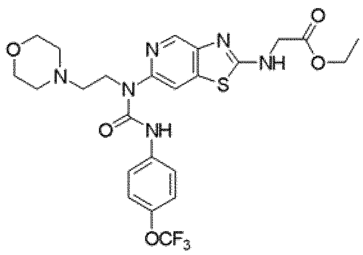 | MS (ESI) m/z:569.4[M+H]$^+$ |
| **44** | 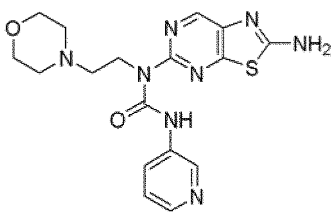 | MS (ESI) m/z:401.2[M+H]$^+$ |
| **45** | 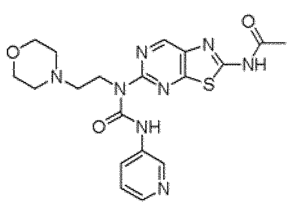 | MS (ESI) m/z:443.1[M+H]$^+$ |

(continued)

| Example | Structural Formula | $^1$H-NMR, MS m/z data |
|---|---|---|
| **46** | 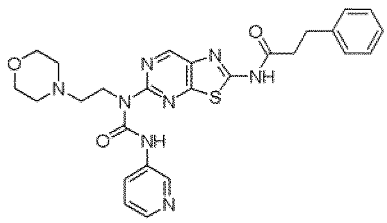 | MS (ESI) m/z:533.0[M+H]$^+$ |
| **47** | 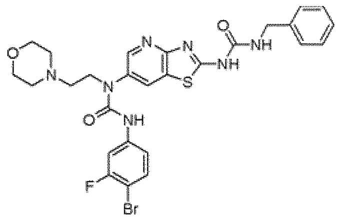 | MS (ESI) m/z:610.5[M+H]$^+$ |

**The compounds of the present invention were studied for the *in vitro* enzyme activity of soluble epoxide hydrolase (sEH), and the results were as follows.**

The benzothiazole skeleton compounds (concentration: 0.1 μM) according to the above formula I of the present invention were tested for the *in vitro* sEH enzyme activity by fluorescence analysis. The reference t-AUCB was home-made by the research group (Hwang S H, Wecksler A T, Zhang G, et al. Synthesis and biological evaluation of sorafenib- and regorafenib-like sEH inhibitors. Bioorganic & Medicinal Chemistry Letters, 2013, 23(13): 3732-3737.).

12 μL of sEH enzyme (human recombinant, 0.5 mg/mL, Cayman Chemical) was diluted with 588 μL of buffer (25 mM *bis*-Tris, pH 7.0). The fluorescent substrate was PHOME (cyano-(6-methoxy-naphthalen-2-yl)-methyl (3-phenyl-oxiranyl)-acetate) in DMSO (10 μM, Cayman Chemical). The compound to be tested was dissolved in 100% DMSO at a concentration of 4 μM. 185 μL of buffer (25 mM *bis*-Tris, pH 7.0), 5 μL of compound solution, and 5 μL of diluted sEH enzyme were added to the wells of a 96-well plate, and finally 5 μL of substrate solution was added to initiate the reaction. After incubating at 37 °C for 15 min, the fluorescence signal was measured with a microplate reader (excitation wavelength 330 nm, emission wavelength 465 nm). The background group and the full-active group were set at the same time, wherein the background group excluded the compound and the enzyme (replaced by DMSO and buffer, respectively), and the full-active group excluded the compound (replaced by DMSO). The inhibition was calculated from the data of the full-active group ($F_0$) and the compound group ($F_1$) after deducting the fluorescence signal of the background group:

$$\text{Inhibition (\%)} = (F_0 - F_1) / (F_0) \times 100.$$

The *in vitro* sEH enzyme activity test results of the compounds of Examples were shown in Table 2.

**Table 2**

| Example compounds | sEH %inhibition, @ 0.1μM |
|---|---|
| **Example 1** | 76.1 |
| **Example 2** | 11.7 |
| **Example 3** | 32.9 |
| **Example 4** | 77.3 |
| **Example 5** | 47.0 |
| **Example 6** | 2.15 |
| **Example 7** | 59.9 |
| **Example 8** | 19.2 |
| **Example 9** | 28.2 |

(continued)

| Example compounds | sEH %inhibition, @ 0.1μM |
|---|---|
| **Example 10** | 2.16 |
| **Example 11** | 22.0 |
| **Example 12** | 68.7 |
| **Example 13** | 63.9 |
| **Example 14** | 16.2 |
| **Example 15** | 39.0 |
| **Example 16** | 79.7 |
| **Example 17** | 70.3 |
| **Example 18** | 84.7 |
| **Example 19** | 76.6 |
| **Example 20** | 75.2 |
| **Example 21** | 86.2 |
| **Example 22** | 67.5 |
| **Example 23** | 43.7 |
| **Example 24** | 28.4 |
| **Example 25** | 70.4 |
| **Example 26** | 36.5 |
| **Example 27** | 79.4 |
| **Example 28** | 90.5 |
| **Example 29** | 69.0 |
| **Example 30** | 27.5 |
| **Example 31** | 70.9 |
| **Example 32** | 77.6 |
| **Example 33** | 15.8 |
| **Example 34** | 32.5 |
| **Example 35** | 10.3 |
| **Example 36** | 45.6 |
| **Example 37** | 66.8 |
| **Example 38** | 57.2 |
| **Example 39** | 41.5 |
| **Example 40** | 43.0 |
| **Example 41** | 34.7 |
| **Example 42** | 52.1 |
| **Example 43** | 78.2 |
| **Example 44** | 55.9 |
| **Example 45** | 45.2 |
| **Example 46** | 67.9 |
| **Example 47** | 32.5 |

(continued)

| Example compounds | sEH %inhibition, @ 0.1μM |
|---|---|
| **t-AUCB** | 68.7 |

**Some compounds of the present invention were studied for the *in vitro* $IC_{50}$ of the soluble epoxide hydrolase (sEH), and the results were as follows.**

The benzothiazole skeleton compounds according to the above formula I of the present invention were tested for the *in vitro* sEH enzyme activity by fluorescence analysis. The reference t-AUCB was home-made by the research group.

12 μL of sEH enzyme (human recombinant, 0.5 mg/mL, Cayman Chemical) was diluted with 588 μL of buffer (25 mM *bis*-Tris, pH 7.0). The fluorescent substrate was PHOME (cyano-(6-methoxy-naphthalen-2-yl)-methyl (3-phenyl-oxiranyl)-acetate) in DMSO (10 μM, Cayman Chemical). The compound to be tested was dissolved in 100% DMSO at a concentration of 4μM, and successively diluted into solutions with different concentrations (2μM, 1μM, 0.5μM, 0.25μM, 0.04μM, 0.02μM, 0.004μM, and 0.0004μM). The inhibitions at different concentrations for the compound were respectively measured in accordance with the inhibition assay, to calculate $IC_{50}$.

The sEH enzyme $IC_{50}$ activity data for some Examples were shown in Table 3.

**Table 3**

| Examples | $IC_{50}$ (nM) |
|---|---|
| **Ex. 1 compound** | 4.8 |
| **Ex. 4 compound** | 122 |
| **Ex. 7 compound** | 9.2 |
| **Ex. 12 compound** | 4.1 |
| **Ex. 13 compound** | 2.8 |
| **Ex. 16 compound** | 0.25 |
| **Ex. 18 compound** | 6.7 |
| **Ex. 27 compound** | 0.02 |
| **Ex. 29 compound** | 2.6 |
| **Ex. 25 compound** | 27.1 |
| **Ex. 30 compound** | 1.9 |
| **Ex. 40 compound** | 0.11 |
| **t-AUCB** | 6.8 |

The preliminary *in vitro* sEH enzyme activity test results showed that, the compounds of general formula I as claimed in the present invention had a good inhibitory activity on sEH, and some compounds were equivalent to or better than the positive control drug t-AUCB.

**Example 48 Measurement of anti-fibrosis effect**

Male C57BLl6J mice (6 to 8 weeks old) (SPF (Beijing) Biotechnology Co., Ltd.) were divided into 3 groups: sham group, unilateral ureteral obstruction (UUO) group, and UUO + Ex. 16 compound group. After the mice were anesthetized, the left ureter was ligated and cut to create a model. In the sham group, the ureter was only isolated without ligation. The experimental animals were administrated 72 hours before modeling, and the aqueous solution of Ex. 16 compound (10% PEG400, 10% Tween 80, concentration: 9 mg/mL) was intraperitoneally injected at a dose of 30 $mg \cdot kg^{-1} \cdot d^{-1}$; and the sham group and UUO group were intraperitoneally injected with an equal volume of blank solvent. 7 days after modeling, the animals were sacrificed, and samples were taken for testing. The results showed that, the compound of Example 16 could maintain the level of EETs in mouse kidney tissue (Kim J, Imig JD, Yang J, et al. Inhibition of soluble epoxide hydrolase prevents renal interstitial fibrosis and inflammation. Am J Physiol Renal Physiol, 2014, 307(8): F971-F980.), reduce the collagen deposition, and decrease the fibrotic areas. The results were shown in Figs.

1 and 2.

**Example 49 Measurement of anti-inflammatory effect**

Male KM mice (6 to 8 weeks old) (SPF (Beijing) Biotechnology Co., Ltd.) were subcutaneously with 50μL of 1% λ-carrageenan saline solution at planta to prepare the model of inflammation and swelling (Posadas I, Bucci M, Roviezzo F, et al. Carrageenan-induced mouse paw oedema is biphasic, age-weight dependent and displays differential nitric oxide cyclooxygenase-2 expression. British Journal of Pharmacology, 2004, 142(2):331-338.). 1 hour in advance, the mice were intraperitoneally injected with the solutions of the compounds of Examples 12, 18, 27, and 40 (10% PEG 400, 10% Tween 80) or the blank solvent, respectively. Taking the initial paw thickness of the mice before modeling as 100%, the changes in the paw thickness of the mice were measured. The results showed that, the compounds could significantly inhibit the carrageenan-induced paw swelling in mice, and the effects were better than that of celecoxib. The results were shown in Fig. 3.

**Example 50 Measurement of brain injury protection**

45 male SD rats (6 to 8 weeks old) (SPF (Beijing) Biotechnology Co., Ltd.) were divided into the sham group, blank solvent group, and dosing group. 72 hours after intraperitoneally injecting the compound of Example 12 or blank solvent, a traumatic brain injury model was established using the modified Feeney method (Marmarou A, Foda MA, van den Brink W, et al., A new model of diffuse brain injury in rats. Part I: Pathophysiology and biomechanics. Journal of Neurosurgry, 1994, 80(2):291 to 300.). 23 hours after model establishment, three mice in each group were injected with Evans blue solution through the tail vein, and after 1 hour of circulation, the mice were decapitated and the brain was taken out to determine the content of Evans blue (EB), so as to study the permeability. Another three rats were taken to measure the water content in brain. The results showed that, the compound of Example 12 could reduce the water content and permeability in brain. The results were shown in Table 4.

**Table 4**

| Group | Water content in brain (%) | EB content (μg/g) |
|---|---|---|
| **Sham group** | 77.46±0.57 * | 9.26± 1.24 * |
| **Blank solvent group** | 82.45±1.47 | 30.86±3.33 |
| **Example 12 (30mg/kg)** | 79.46±0.68 * | 25.23±1.63 * |
| *: Compared with the blank solvent group, $p < 0.05$ | | |

**Example 51 Measurement of hypoglycemic effect**

Male Balb/c mice (6 to 8 weeks old) (SPF (Beijing) Biotechnology Co., Ltd.) were intraperitoneally injected with streptozotocin STZ (40mg/kg) once a day for 5 consecutive days, to prepare a diabetic model (Like AA, Rossini AA. Streptozotocin-induced pancreatic insulitis: new model of diabetes mellitus. Science, 1976, 193(4251):415-7). The mice after modeling were intraperitoneally injected with the solution of compound of Example 12 once a day. After two weeks, the blood glucose of the dosing group was significantly lower than that of the model group, and the glucose tolerance was higher than that of the model group. The results were shown in Fig. 4.

**Example 52 Improvement effect on diabetic retinopathy**

Male Wistar rats (180 to 200 g) (Experimental Animal Center, Hebei Medical University) were intraperitoneally injected with streptozotocin STZ (60mg/kg) to prepare a diabetic model, and were fed for 3 months to induce a retinopathy model (Miyamoto K, Khosrof S, Bursell SE, et al. Prevention of leukostasis and vascular leakage in streptozotocin-induced diabetic retinopathy via intercellular adhesion molecule-1 inhibition. Proc Natl Acad Sci U S A, 1999, 96(19): 10836-10841). The compound of Example 40 was dissolved in 10% hydroxypropyl-β-cyclodextrin solution, to be prepared into eye drops with a content of 0.1% for the compound of Example 40. The rats were topically administrated in eyes every day. After 6 months, the rats developed non-proliferative diabetic retinopathy symptoms of increased retinal capillary free pericytes and increased vascular leakage, while in Example 40 group these symptoms were significantly alleviated. The results were shown in Fig. 5.

**Example 53 Measurement of analgesic effect**

Male SD rats (6 to 8 weeks old) (SPF (Beijing) Biotechnology Co., Ltd.) were subcutaneously injected with 50μL of 1% λ-carrageenan saline solution at planta to prepare an inflammatory pain model (Rose TE, Morisseau C, Liu JY, et al. 1-Aryl-3-(1-acylpiperidin-4-yl)urea inhibitors of human and murine soluble epoxide hydrolase: structure-activity relationships, pharmacokinetics, and reduction of inflammatory pain. J Med Chem, 2010, 53(19): 7067-7075). The compounds of Examples 12 and 40 were added to 0.5% sodium carboxymethylcellulose solution and ground to prepare a suspension with a concentration of 15 mg/mL. 1 hour before modeling, the rats were intragastrically administrated. The changes in the paw withdrawal time of rats were measured with a hot plate dolorimeter. The results showed that, the compounds of Examples 12 and 40 could significantly prolong the paw withdrawal time of rats in the hot plate dolorimeter, increase the pain threshold and relieve the pain. The results were shown in Fig. 6.

**Example 54 Measurement of antidepressant effect**

Male KM mice (6 to 8 weeks old) (SPF (Beijing) Biotechnology Co., Ltd.) swam in advance for 10 min, and were injected with lipopolysaccharide (0.5 mg/kg). After 23 hours, the mice were subcutaneously injected with the solutions of the compounds of Examples 12 and 40 or the blank solvent. After 1 hour, the mice were subjected to the forced swimming test for 6 min, and the immobility time (s) in later 4 minute for the mice in each group was recorded. The results showed that, the compounds of Examples 12 and 40 could significantly reduce the immobility time of mice in forced swimming. The results were shown in Fig. 7.

Those skilled in the art should understand that, although the invention is described in details with reference to the above Examples, the invention is not limited to these specific Examples. Based on the methods and technical solutions taught by the invention, those skilled in the art can make appropriate modifications or improvements without departing from the spirit of the invention, and the equivalent embodiments thus obtained are all within the scope of the invention.

## Claims

1. A compound of general formula I or its stereoisomers as well as pharmaceutically acceptable salts, solvates or prodrugs thereof,

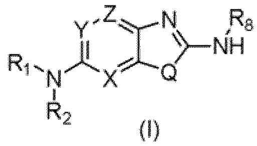

(I)

wherein,

X is CH or N;
Y is CH or N;
Z is CH, N or bond;
Q is O, S, NH or $NCH_3$;
$R_1$ is hydrogen, $-(CH_2)_mNR_3R_4$, $-C(O)NH(CH_2)_nR_5$, $-C(O)(CH_2)_nR_5$ or $-S(O)_2NH(CH_2)_nR_5$;
m is an integer from 2 to 4;
$R_3$ and $R_4$ are same or different, and independently selected from hydrogen, $(C_1$-$C_6)$alkyl, $(C_3$-$C_7)$cycloalkyl, $(C_1$-$C_6)$alkylacyl, $(C_1$-$C_6)$alkoxyl, $(C_2$-$C_6)$alkenyl, and $(C_2$-$C_6)$alkynyl; or
$R_3$ and $R_4$ together with the nitrogen atom to which they are attached form a 4 to 10 membered heterocyclic group or 5 to 10 membered heteroaryl group, wherein the heterocyclic group or heteroaryl group optionally contains 0 to 4 heteroatom(s) selected from N, O, and/or S in addition to the nitrogen atom to which $R_3$ and $R_4$ are attached, the heterocyclic group optionally comprises 0 to 2 carbon-carbon double bond(s) or carbon-carbon triple bond(s) in addition to the nitrogen atom to which $R_3$ and $R_4$ are attached, and the heterocyclic group or heteroaryl group is optionally substituted by 1 to 3 same or different $R_6$;
$R_2$ is hydrogen, $-C(O)NH(CH_2)_nR_5$, $-C(O)(CH_2)_nR_5$, $-S(O)_2NH(CH_2)_nR_5$ or $-(CH_2)_mNR_3R_4$;
$R_1$ and $R_2$ are not hydrogen at the same time;
n is an integer from 0 to 4;
$R_5$ is hydrogen, $(C_1$-$C_6)$alkyl, $(C_3$-$C_{10})$cycloalkyl, $(C_6$-$C_{10})$aryl or 5 to 10 membered heteroaryl group, wherein

the heteroaryl group contains 1 to 3 heteroatom(s) selected from N, O or S, and the aryl or heteroaryl group is optionally substituted by 1 to 3 same or different $R_7$;
$R_8$ is hydrogen, $-C(O)(CH_2)_qR_9$, $-(CH_2)_qC(O)OR_9$ or $-C(O)NH(CH_2)qR_9$;
$R_9$ is $(C_1-C_6)$alkyl, $(C_{3-}C_7)$cycloalkyl, $(C_6-C_{10})$aryl or 5 to 10 membered heteroaryl group, wherein the aryl or heteroaryl group is optionally substituted by 1 to 3 same or different $R_{10}$;
q is an integer from 0 to 4;
$R_6$, $R_7$, and $R_{10}$ are independently 1 to 3 same or different substituent(s) selected from hydrogen, hydroxyl, halogen, nitro, amino, cyano, azido, mercapto, $(C_1-C_6)$alkyl, $(C_1-C_6)$alkoxyl, $(C_1-C_6)$alkyl or $(C_1-C_6)$alkoxyl optionally substituted by hydroxyl, amino or halogen, $(C_1-C_6)$alkylthiol, allyl, amino substituted by mono or di($C_1-C_6$ alkyl), $(C_1-C_6)$alkylamido, free, salified, esterified, and amidated carboxyl, $(C_1-C_6)al_k$ylsulfinyl, $(C_1-C_6)$alkylsulfonyl, $(C_1-C_6)$alkylacyl, carbamoyl or carbamoyl substituted by mono or di($C_1-C_6$ alkyl).

2. The compound of general formula **I** or its stereoisomers as well as pharmaceutically acceptable salts, solvates or prodrugs thereof according to claim 1,
wherein,

Z is CH;
$R_1$ is hydrogen or $-(CH_2)_mNR_3R_4$;
$R_2$ is hydrogen, $-C(O)NH(CH_2)_nR_5$ or $-C(O)(CH_2)_nR_5$;
$R_1$ and $R_2$ are not hydrogen at the same time;
$R_3$ and $R_4$ are same or different, and independently selected from hydrogen, $(C_1-C_6)$alkyl, $(C_{3-}C_7)$cycloalkyl, $(C_1-C_6)$alkylacyl, $(C_1-C_6)$alkoxyl, $(C_2-C_6)$alkenyl, and $(C_2-C_6)$alkynyl; or
$R_3$ and $R_4$ together with the nitrogen atom to which they are attached form a 4 to 10 membered heterocyclic group, wherein the heterocyclic group optionally contains 1 to 4 heteroatom(s) selected from N, O, and/or 5 in addition to the nitrogen atom to which $R_3$ and $R_4$ are attached, and optionally substituted by 1 to 3 same or different $R_6$.

3. The compound of general formula **I** or its stereoisomers as well as pharmaceutically acceptable salts, solvates or prodrugs thereof according to claim 1 or 2,
$R_5$ is $(C_3-C_{10})$cycloalkyl, phenyl or a 5 to 10 membered heteroaryl group containing 1 to 3 heteroatom(s) selected from N, O or S; more preferably $(C_{3-}C_7)$cycloalkyl, phenyl, indolyl, quinolyl, pyridyl, pyrimidinyl, furyl, thienyl or pyrrolyl, wherein aryl or heteroaryl group is optionally substituted by 1 to 3 same or different $R_7$.

4. The compound of general formula **I** or its stereoisomers as well as pharmaceutically acceptable salts, solvates or prodrugs thereof according to any of claims 1 to 3,
wherein,

$R_1$ is hydrogen or $-(CH_2)_mNR_3R_4$;
$R_3$ and $R_4$ are same or different, and independently selected from hydrogen, $(C_1-C_6)$alkyl, and $(C_3-C_7)$cycloalkyl or together with the nitrogen atom to which they are attached form

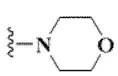, 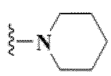, 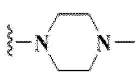, 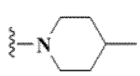, 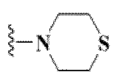, 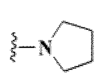, 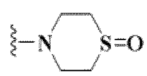 or 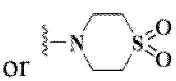;

$R_2$ is hydrogen, $-C(O)NH(CH_2)_nR_5$ or $-C(O)(CH_2)_nR_5$.

5. The compound of general formula **I** or its stereoisomers as well as pharmaceutically acceptable salts, solvates or prodrugs thereof according to any of claims 1 to 4,
$R_9$ is phenyl optionally substituted by 1 to 3 same or different $R_{10}$.

6. The following compound of general formula I or its stereoisomers as well as pharmaceutically acceptable salts, solvates or prodrugs thereof:

1-(2-aminobenzo[*d*]thiazol-6-yl)-3-(4-chlorophenyl)urea;
1-(2-aminobenzo[*d*]thiazol-6-yl)urea;
1-(2-aminobenzo[*d*]thiazol-6-yl)-3-phenylurea;
1-(2-aminobenzo[*d*]thiazol-6-yl)-3-[(3s,5s,7s)-adamantan-1-yl]urea;

1-(2-aminobenzo[*d*]thiazol-6-yl)-3-(4-chlorobenzyl)urea;
1-(2-aminobenzo[*d*]thiazol-6-yl)-3-cyclohexylurea;
1,1'-(benzo[*d*]thiazol-2,6-diyl)bis[3-(4-chlorophenyl)urea];
1-(2-aminobenzo[*d*]thiazol-6-yl)-1-[2-(4-morpholinyl)ethyl]-3-(4-nitrophenyl)urea;
1-(2-aminobenzo[*d*]thiazol-6-yl)-1-[2-(4-morpholinyl)ethyl]-3-(4-acetylphenyl)urea;
1-(2-aminobenzo[*d*]thiazol-6-yl)-1-[2-(4-morpholinyl)ethyl]-3-(4-acetamidophenyl)urea;
N-(2-aminobenzo[*d*]thiazol-6-yl)-2-(4-chlorophenyl)-N-[2-(4-morpholinyl)ethyl]acetamide;
1-(2-aminobenzo[*d*]thiazol-6-yl)-1-[2-(4-morpholinyl)ethyl]-3-(4-chlorophenyl)urea;
1-(2-aminobenzo[*d*]thiazol-6-yl)-1-[2-(4-morpholinyl)ethyl]-3-(4-trifluoromethylphenyl)urea;
1-(2-aminobenzo[*d*]thiazol-6-yl)-1-[2-(4-morpholinyl)ethyl]-3-(pyridin-2-yl)urea;
1-(2-aminobenzo[*d*]thiazol-6-yl)-1-[2-(4-morpholinyl)ethyl]-3-(naphthalen-1-yl)urea;
1-(2-aminobenzo[*d*]thiazol-6-yl)-1-[2-(4-morpholinyl)ethyl]-3-(3-bromo-4-fluorophenyl)urea;
1-(2-aminobenzo[*d*]thiazol-6-yl)-1-[2-(4-morpholinyl)ethyl]-3-(4-trifluoromethoxyphenyl)urea;
1-(2-aminobenzo[*d*]thiazol-6-yl)-1-[2-(4-morpholinyl)etliyl]-3-(4-ethylphenyl)urea;
1-(2-aminobenzo[*d*]thiazol-6-yl)-l-[2-(4-morpholinyl)ethyl]-3-(4-isopropoxyphenyl)urea;
1-(2-aminobenzo[*d*]thiazol-6-yl)-1-[2-(4-morpholinyl)ethyl]-3-(4-isopropylphenyl)urea;
1-(2-aminobenzo[*d*]thiazol-6-yl)-1-[2-(N,N-diethylamino)ethyl]-3-(4-chlorophenyl)urea;
1-(2-aminobenzo[*d*]thiazol-6-yl)-1-[2-(4-methylpiperidin-1-yl)ethyl]-3-(4-chlorophenyl)urea;
1-(2-aminobenzo[*d*]thiazol-6-yl)-1-[2-(4-methylpiperazin-1-yl)ethyl]-3-(4-trifluoromethylphenyl)urea;
1-(2-aminobenzo[*d*]thiazol-6-yl)-1-[2-(pyrrolidin-1-yl)ethyl]-3-(4-chlorophenyl)urea;
1-(2-aminobenzo[*d*]thiazol-6-yl)-1-[2-(N,N-dimethylamino)ethyl]-3-(4-chlorophenyl)urea;
1-(2-aminobenzo[*d*]thiazol-6-yl)-1-[3-(4-morpholinyl)propyl]-3-(4-chlorophenyl)urea;
1-(2-phenylacetamidobenzo[*d*]thiazol-6-yl)-1-[2-(4-morpholinyl)ethyl]-3-(4-chlorophenyl)urea;
1-[2-(2-ethoxyformamido)benzo[*d*]thiazol-6-yl]-1-[2-(4-morpholinyl)ethyl]-3-(4-chlorophenyl)urea;
1-{[2-(2-ethoxyformyl)ethylamino-1-yl]benzo[*d*]thiazol-6-yl}-1 -[2-(4-morpholinyl)ethyl]-3-(4-chlorophenyl)urea;
1-(2-acetamidobenzo[*d*]thiazol-6-yl)-1-[2-(4-morpholinyl)ethyl]-3-(4-chlorophenyl)urea;
Tert-butyl (6-{2-(4-chlorophenyl)-N-[2-(4-morpholinyl)ethyl]acetamido}benzo[*d*]thiazol-2-yl)carbamate;
1-(2-phenylformamidobenzo[*d*]thiazol-6-yl)-1-[2-(4-morpholinyl)ethyl]-3-(4-chlorophenyl)urea;
1-(2-phenylpropionamidobenzo[*d*]thiazol-6-yl)-1-[2-(4-morpholinyl)ethyl]-3-(4-chlorophenyl)urea;
1-(2-phenylacetamidobenzo[*d*]thiazol-6-yl)-1-[2-(4-morpholinyl)ethyl]-3-(4-trifluoromethylphenyl)urea;
1-(2-aminobenzo[*d*]oxazol-6-yl)-1-[2-(4-morpholinyl)ethyl]-3-(4-methylphenyl)urea;
1-(2-acetamidobenzo[*d*]oxazol-6-yl)-1-[2-(4-morpholinyl)ethyl]-3-(4-methylphenyl)urea,
1-(2-phenylacetamidobenzo[*d*]oxazol-6-yl)-1-[2-(4-morpholinyl)ethyl]-3-(4-methylphenyl)urea;
1-(2-aminothiazo[5,4-*b*]pyridin-5-yl)-3-(4-fluorophenyl)-1-[2-(4-morpholinyl)ethyl]urea-,
1-[2-(ethylamino)thiazo[5,4-*b*]pyridin-5-yl]-1-[2-(4-morpholinyl)ethyl]-3-(4-fluorophenyl)urea;
1-{2-[3-(4-chlorophenyl)ureido]benzo[*d*]thiazol-6-yl}-1-[2-(4-morpholinyl)ethyl]-3-(4-chlorophenyl)urea,
1-(2-aminothiazo[4,5-*c*]pyridin-6-yl)-1-[2-(4-morpholinyl)ethyl]-3-(4-trifluoromethoxyphenyl)urea;
1-(2-cyclopropylformamidothiazo[4,5-*c*]pyridin-6-yl)-1-[2-(4-morpholinyl)ethyl]-3-(4-trifluoromethoxyphenyl)urea;
1-{[2-(2-ethoxyformyl)ethylamino-1-yl]thiazo[4, 5-c]pyridin-6-yl 1- 1-[2-(4-morpholinyl)ethyl]-3-(4-trifluoromethoxyphenyl)urea;
1-(2-aminothiazo[5,4-*d*]pyrimidin-5-yl)-1-[2-(4-morpholinyl)ethyl]-3-(pyridin-3-yl)urea;
1-(2-acetamidothiazo[5,4-*d*]pyrimidin-5-yl)-1-[2-(4-morpholinyl)ethyl]-3-(pyridin-3-yl)urea;
1-(2-phenylpropionamidothiazo[5,4-*d*]pyrimidin-5-yl)-1-[2-(4-morpholinyl)ethyl]-3-(pyridin-3-yl)urea;
1-[2-(3-benzylureido)thiazo[4,5-*d*]pyridin-6-yl]-1-[2-(4-morpholinyl)ethyl]-3-(4-bromo-3-fluorophenyl)urea.

**7.** The compound of general formula **I** or its stereoisomers as well as pharmaceutically acceptable salts, solvates or prodrugs thereof, wherein the pharmaceutically acceptable salt is a salt formed with an acid, and the acid is selected from: hydrochloric acid, hydrobromic acid, hydrofluoric acid, sulfuric acid, phosphoric acid, nitric acid, formic acid, acetic acid, propionic acid, oxalic acid, malonic acid, succinic acid, fumaric acid, maleic acid, lactic acid, malic acid, tartaric acid, citric acid, picric acid, methanesulfonic acid, ethanesulfonic acid, toluenesulfonic acid, benzenesulfonic acid, naphthalenesulfonic acid, trifluoroacetic acid or aspartic acid.

**8.** A pharmaceutical composition comprising the compound of general formula **I** or its stereoisomers as well as pharmaceutically acceptable salts, solvates or prodrugs thereof, and a pharmaceutically acceptable carrier.

**9.** Use of the compound of general formula **I** or its stereoisomers as well as pharmaceutically acceptable salts, solvates or prodrugs thereof according to any of claims 1-7 or the pharmaceutical composition according to claim 8 in the

preparation of a medicament for treating and/or preventing a sEH-mediated disease.

**10.** The use according to claim 9, **characterized in that**, the sEH-mediated disease includes inflammatory diseases, cardiovascular and cerebrovascular diseases, diabetes, diabetic complications, diabetes-related diseases, fibrotic diseases, neurological and mental diseases, pains, and ulcerative diseases.

**11.** The use according to claim 10, **characterized in that**, the inflammatory diseases include: inflammatory liver disease, inflammatory kidney disease, inflammatory lung disease, inflammatory brain disease, pancreatitis, arthritis, soft tissue inflammation, bone tissue inflammation, and vascular inflammation; the cardiovascular and cerebrovascular diseases include: hypertension, myocardial infarction, heart failure, coronary heart disease, cardiovascular arteriosclerosis, cerebral ischemic stroke, and cerebral hemorrhagic stroke; the diabetes includes: type I diabetes and type II diabetes; the diabetic complications include: diabetic retinopathy, diabetes-related uveitis, diabetic cataract, diabetic nephropathy, diabetic dermopathy, and diabetic peripheral neuropathy; the diabetes-related diseases include: hyperlipidemia, hyperuricemia and gout, obesity, and metabolic syndrome; the fibrotic disease include: pulmonary fibrosis, hepatic fibrosis, myocardial fibrosis, and renal fibrosis; the neurological and mental diseases include: Alzheimer’s disease, epilepsy, Parkinson’s disease, schizophrenia, dysphrenia, depression, and neurasthenia; the pain diseases include: neuropathic pain, inflammatory pain, neoplastic pain and mixed pain; and the ulcerative diseases include: gastric ulcer, duodenal ulcer, ulcerative colitis, corneal ulcer, and oral ulcer.

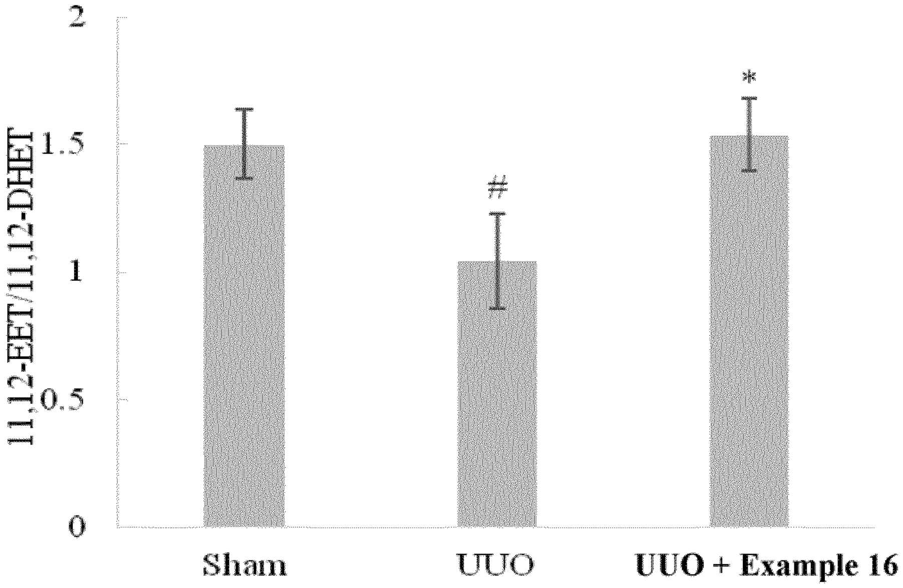
2
1.5
1
0.5
0
11,12-EET/11,12-DHET
*
#
Sham
UUO
UUO + Example 16

Fig. 1

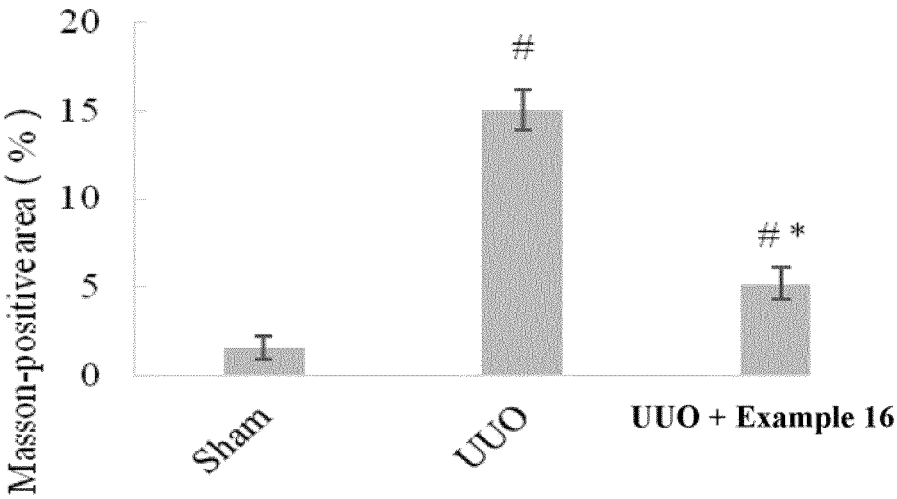
20
15
10
5
0
Masson-positive area ( % )
#
# *
Sham
UUO
UUO + Example 16

Fig. 2

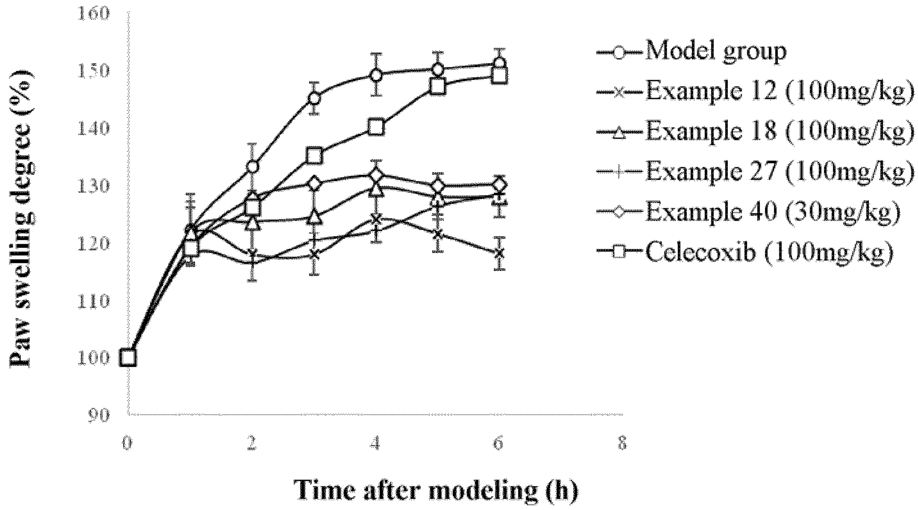
Paw swelling degree (%)
160
150
140
130
120
110
100
90
0
2
4
6
8
Time after modeling (h)
Model group
Example 12 (100mg/kg)
Example 18 (100mg/kg)
Example 27 (100mg/kg)
Example 40 (30mg/kg)
Celecoxib (100mg/kg)

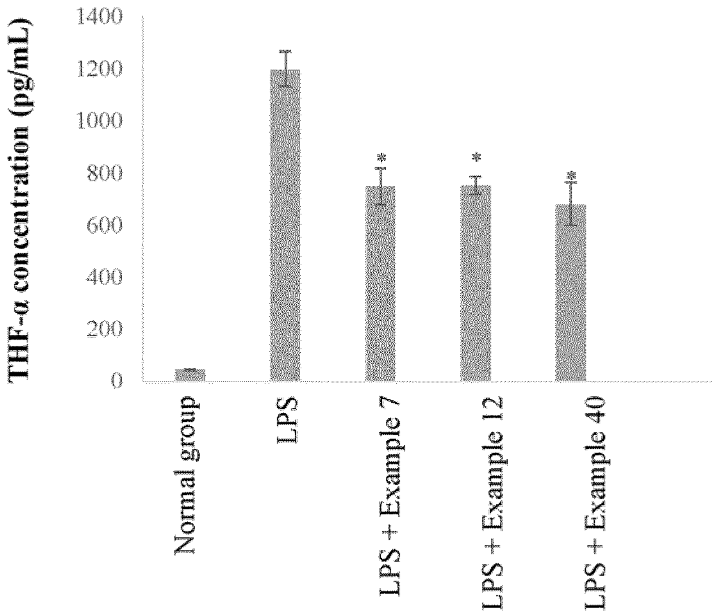
THF-α concentration (pg/mL)
1400
1200
1000
800
600
400
200
0
*
*
*
Normal group
LPS
LPS + Example 7
LPS + Example 12
LPS + Example 40

Fig. 3

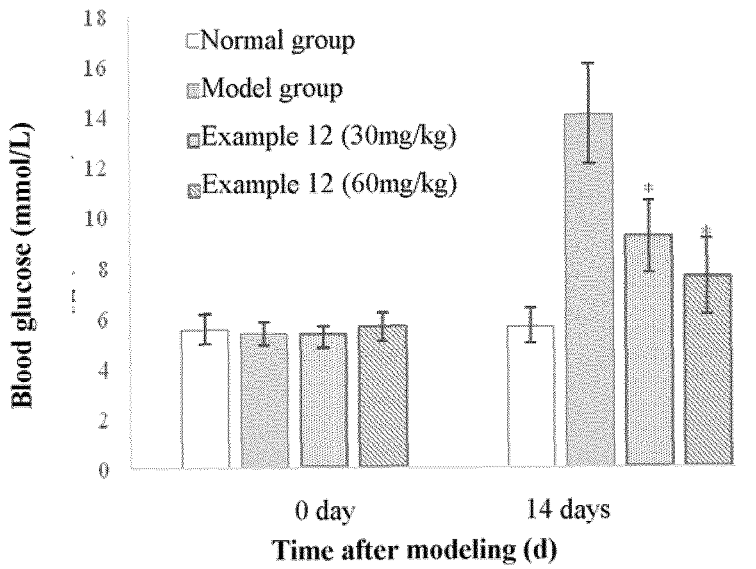
Normal group
Model group
Example 12 (30mg/kg)
Example 12 (60mg/kg)
Blood glucose (mmol/L)
0 day
14 days
Time after modeling (d)

Fig. 4

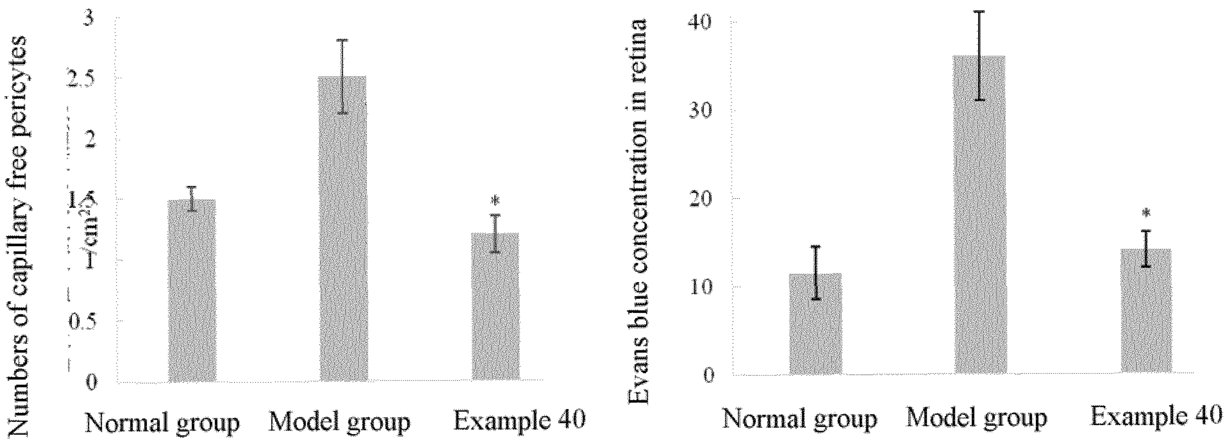
Numbers of capillary free pericytes /cm²
Normal group
Model group
Example 40
Evans blue concentration in retina
Normal group
Model group
Example 40

Fig. 5

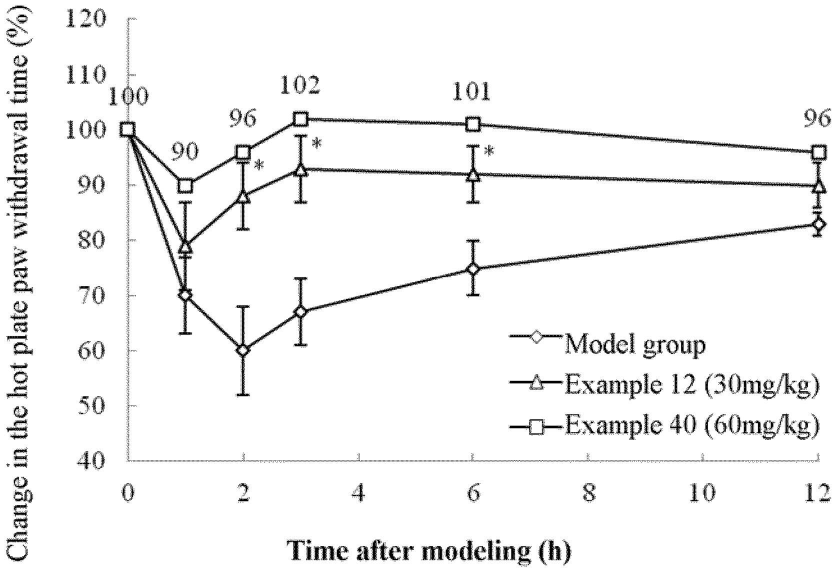
Change in the hot plate paw withdrawal time (%)
120
110
100
90
80
70
60
50
40
0
2
4
6
8
10
12
100
90
96
102
101
96
*
*
*
Model group
Example 12 (30mg/kg)
Example 40 (60mg/kg)
Time after modeling (h)

**Fig. 6**

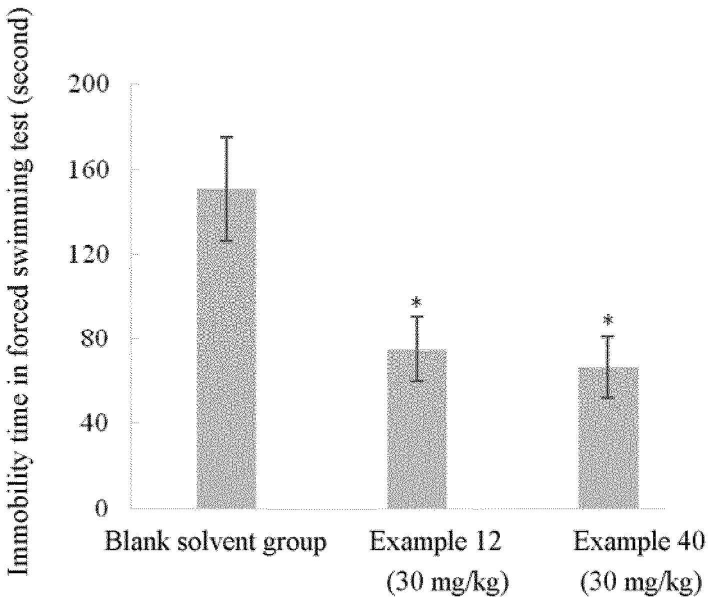
Immobility time in forced swimming test (second)
200
160
120
80
40
0
*
*
Blank solvent group
Example 12
(30 mg/kg)
Example 40
(30 mg/kg)

**Fig. 7**

## INTERNATIONAL SEARCH REPORT

International application No.
**PCT/CN2021/097571**

**A. CLASSIFICATION OF SUBJECT MATTER**

C07D 277/82(2006.01)i; C07D 417/12(2006.01)i; C07D 513/04(2006.01)i; A61K 31/5377(2006.01)i; A61K 31/496(2006.01)i; A61K 31/4439(2006.01)i; A61P 29/00(2006.01)i; A61P 9/00(2006.01)i; A61P 3/10(2006.01)i; A61P 25/18(2006.01)i; A61P 25/00(2006.01)i; A61P 1/04(2006.01)i; A61P 27/02(2006.01)i

According to International Patent Classification (IPC) or to both national classification and IPC

**B. FIELDS SEARCHED**

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

CNABS; CNTXT; CJFD; DWPI; SIPOABS; WOTXT; USTXT; EPTXT; CNKI; PATENTICS; 万方; 超星读秀; ISI-Web of Science; STN-registry; STN-caplus: 沈阳药科大学, 河北科技大学, 赵燕芳, 高子彬, 韩雨霏, 李硕, 侯云雷, 张惠敏, 徐思聪, 孙艳平, 秦铭泽, 孙勇军, 刘亚婧, 宫平, 苯并噻唑, 脲, 环氧化物水解酶, 炎症, 心脑血管疾病, 糖尿病, 纤维化疾病, 精神疾病, 疼痛, 溃疡, Epoxide Hydrolase, sEH, +benzothiazolyl+, Benzothiazole, Urea, Inflammation, Inflammatory, Cardiovascular System, Nervous System, Diabetes, Fibrosis, Ulcers, structural formula (I)

**C. DOCUMENTS CONSIDERED TO BE RELEVANT**

| Category* | Citation of document, with indication, where appropriate, of the relevant passages | Relevant to claim No. |
|---|---|---|
| X | WO 2006066795 A1 (4SC AG et al.) 29 June 2006 (2006-06-29)<br>description page 56 line 5, page 58 lines 17-26 | 1-5 |
| X | BOUANANE Abdelhakim et al. "Thiocyanation des Dianilines. I. Resultats Experimentaux" *Bulletin de la Societe Chimique de France,* No. 3-4, 31 December 1974 (1974-12-31), ISSN: 0037-8968,<br>page 644 compound 6 | 1-5 |
| X | "RN: 1350153-09-1"<br>*REGISTRY enters STN,* 07 December 2011 (2011-12-07), | 1-4 |
| X | "RN: 1350144-44-3"<br>*REGISTRY enters STN,* 07 December 2011 (2011-12-07), | 1-4 |
| X | "RN: 1349444-89-8"<br>*REGISTRY enters STN,* 06 December 2011 (2011-12-06), | 1-4 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **21 July 2021** | **24 August 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/097571**

**C. DOCUMENTS CONSIDERED TO BE RELEVANT**

| Category* | Citation of document, with indication, where appropriate, of the relevant passages | Relevant to claim No. |
|---|---|---|
| X | "RN: 1349045-32-4"<br>*REGISTRY enters STN,* 05 December 2011 (2011-12-05), | 1-4 |
| A | WO 2009035951 A2 (ARETE THERAPEUTICS INC et al.) 19 March 2009 (2009-03-19)<br>description paragraph [0006], claims 1, 36-41 | 1-11 |
| A | CN 108349955 A (UNIVERSITY OF BARCELONA) 31 July 2018 (2018-07-31)<br>entire document | 1-11 |
| A | WO 2019243414 A1 (UNIV BARCELONA) 26 December 2019 (2019-12-26)<br>entire document | 1-11 |
| A | CN 101679258 A (ARETE THERAPEUTICS INC.) 24 March 2010 (2010-03-24)<br>entire document | 1-11 |
| A | WO 2018077898 A1 (UNIV INNSBRUCK et al.) 03 May 2018 (2018-05-03)<br>entire document | 1-11 |

Form PCT/ISA/210 (second sheet) (January 2015)

**INTERNATIONAL SEARCH REPORT**
**Information on patent family members**

International application No.
**PCT/CN2021/097571**

| Patent document cited in search report | Publication date (day/month/year) | Patent family member(s) | Publication date (day/month/year) |
|---|---|---|---|
| WO 2006066795 A1 | 29 June 2006 | JP 2008524282 A | 10 July 2008 |
| | | EP 1674467 A1 | 28 June 2006 |
| | | EP 1885719 A1 | 13 February 2008 |
| WO 2009035951 A2 | 19 March 2009 | WO 2009035951 A3 | 29 October 2009 |
| CN 108349955 A | 31 July 2018 | BR 112018001816 A2 | 18 September 2018 |
| | | IN 201817007477 A | 06 July 2018 |
| | | ID 201808013 A | 03 August 2018 |
| | | KR 20180030706 A | 23 March 2018 |
| | | EP 3328854 A1 | 06 June 2018 |
| | | AU 2016301027 A1 | 08 March 2018 |
| | | WO 2017017048 A1 | 02 February 2017 |
| | | MX 2018001135 A | 23 May 2018 |
| | | US 2020079786 A1 | 12 March 2020 |
| | | CA 2993882 A1 | 02 February 2017 |
| WO 2019243414 A1 | 26 December 2019 | EP 3584236 A1 | 25 December 2019 |
| | | CA 3104342 A1 | 26 December 2019 |
| | | AU 2019291034 A1 | 14 January 2021 |
| CN 101679258 A | 24 March 2010 | US 2008227780 A1 | 18 September 2008 |
| | | WO 2008112022 A8 | 08 October 2009 |
| | | JP 2010521456 A | 24 June 2010 |
| | | AU 2007349176 A1 | 24 September 2009 |
| | | TW 200837055 A | 16 September 2008 |
| | | BR PI0721451 A2 | 25 March 2014 |
| | | EP 2132176 A1 | 16 December 2009 |
| | | WO 2008112022 A1 | 18 September 2008 |
| | | CA 2680360 A1 | 18 September 2008 |
| | | IN 200903127 P2 | 13 November 2009 |
| | | MX 2009009754 A | 30 September 2009 |
| WO 2018077898 A1 | 03 May 2018 | EP 3315495 A1 | 02 May 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Q. WANG ; W. PANG ; Z. CUI.** *American Journal of physiology-Renal physiology,* 2013, vol. 304, 168-176
- **A. CAUTAUX ; F. ADAM ; C. J. WILLER.** *Joint Bone Spine,* 2005, vol. 72, 359-371
- **C. MORISSEAU ; B. D. HAMMOCK.** *Annual Review of Pharmacology and Toxicology,* 2005, vol. 45, 311-333
- **HWANG S H ; WECKSLER A T ; ZHANG G et al.** Synthesis and biological evaluation of sorafenib- and regorafenib-like sEH inhibitors. *Bioorganic & Medicinal Chemistry Letters,* 2013, vol. 23 (13), 3732-3737
- **KIM J ; IMIG JD ; YANG J et al.** Inhibition of soluble epoxide hydrolase prevents renal interstitial fibrosis and inflammation. *Am J Physiol Renal Physiol,* 2014, vol. 307 (8), F971-F980
- **POSADAS I ; BUCCI M ; ROVIEZZO F et al.** Carrageenan-induced mouse paw oedema is biphasic, age-weight dependent and displays differential nitric oxide cyclooxygenase-2 expression. *British Journal of Pharmacology,* 2004, vol. 142 (2), 331-338
- **MARMAROU A ; FODA MA ; VAN DEN BRINK W et al.** A new model of diffuse brain injury in rats. Part I: Pathophysiology and biomechanics. *Journal of Neurosurgry,* 1994, vol. 80 (2), 291-300
- **LIKE AA ; ROSSINI AA.** Streptozotocin-induced pancreatic insulitis: new model of diabetes mellitus. *Science,* 1976, vol. 193 (4251), 415-7
- **MIYAMOTO K ; KHOSROF S ; BURSELL SE et al.** Prevention of leukostasis and vascular leakage in streptozotocin-induced diabetic retinopathy via intercellular adhesion molecule-1 inhibition. *Proc Natl Acad Sci U S A,* 1999, vol. 96 (19), 10836-10841
- **ROSE TE ; MORISSEAU C ; LIU JY et al.** 1-Aryl-3-(1-acylpiperidin-4-yl)urea inhibitors of human and murine soluble epoxide hydrolase: structure-activity relationships, pharmacokinetics, and reduction of inflammatory pain. *J Med Chem,* 2010, vol. 53 (19), 7067-7075